# EUROPEAN PATENT SPECIFICATION

(11) **EP 3 801 731 B1**
(45) Date of publication and mention of the grant of the patent: **17.01.2024**
(21) Application number: 19734583.8
(22) Date of filing: 11.06.2019
(51) Int. Cl.: A61M 31/00

(54) **APPLICATORS FOR TREATING VAGINAL CONDITIONS**
APPLIKATOREN ZUR BEHANDLUNG VON VAGINALEN ZUSTÄNDEN
APPLICATEURS POUR LE TRAITEMENT D'AFFECTIONS VAGINALES

(30) Priority: 11.06.2018 US 201862683162 P; 11.06.2018 US 201862683153 P; 11.06.2018 US 201862683145 P; 11.06.2018 US 201862683140 P; 11.06.2018 US 201862683177 P; 11.06.2018 US 201862683159 P; 11.06.2018 US 201862683131 P
(43) Date of publication of application: 14.04.2021
(73) Proprietor: The Procter & Gamble Company, Cincinnati, OH 45202 (US)
(72) Inventor: WANG, Samantha, Chen-Yee, Cincinnati, Ohio 45202 (US); BRESLIN, Nery, Vanesa, Cincinnati, Ohio 45202 (US); WILSONHOUCK, Kyra, L., Cincinnati, Ohio 45202 (US); SMITH, Christopher, Lawrence, Cincinnati, OH 45202 (US); LEON, Jessica, Elizabeth, Cincinnati, OH 45202 (US); NACDIMEN, Hali, Michelle, Cincinnati, OH 45202 (US); HODGDON, Travis, Kyle, Cincinnati, OH 45202 (US); SZMURIGA-WINFIELD, Adrienne, Michelle, Cincinnati, OH 45202 (US)
(74) Representative: P&G Patent Germany
(86) International application number: PCT/US2019/036496
(87) International publication number: WO 2019/241210

(56) References cited:
- WO-A1-2006/014572
- US-A- 3 054 403
- US-A- 5 045 058
- US-A1- 2003 088 217
- US-A1- 2008 161 752

## Description

### TECHNICAL FIELD

The present disclosure generally relates to an applicator for administering a composition for treating one or more of vaginal dryness, vaginal irritation, vaginal itch, vaginal infection, vaginal odor, vaginal chafing, dyspareunia, and/or vaginal atrophy, and more specifically, to an applicator comprising a reservoir and a vaginal care composition stored within the reservoir. Disclosed are further (not claimed) methods of using such an applicator.

### BACKGROUND

Estimates indicate that by 2030 there will be about 1.2 billion menopausal and post-menopausal women in the world. Given that the average age at which menopause occurs has remained the same and that life expectancy among women has generally increased, the number of post-menopausal women is expected to grow. As such, there is increasing concern surrounding the conditions and symptoms experienced by peri-menopausal, menopausal, post-menopausal women, and the need for treatment therefore is growing as well.

As is well known, menopause is associated with a decrease in estrogen production. Decreased estrogen levels may result in changes to both the internal and external genitalia, including vaginal atrophy and a thinning of the vaginal and urethral mucous membrane, a loss in vaginal elasticity, and a reduction in gland secretion, which may be accompanied by a decrease in tissue hydration. Some symptoms of decreased estrogen levels may include vaginal infections, irritation, burning, dryness, itching, odor, chafing, atrophy, and pain during sexual intercourse (dyspareunia), thus, greatly impacting a woman's quality of life. For example, women may experience feelings of isolation, fear, resignation, anger, and a loss of libido and intimacy as a result. In addition to menopause, women may also experience a drop in estrogen levels or fluctuating hormones during breastfeeding, breast cancer hormonal treatment, and after surgical removal of the ovaries, pelvic radiation therapy for cancer, and chemotherapy.

There are a variety of solutions that have been proposed to address one or more of the above-described vaginal conditions and symptoms. Prescription-based remedies have included hormone replacement therapy, which can include an estrogen supplement with or without progesterone. In some instances, the hormonal therapies may be applied deep within the vaginal canal by plunger type applicators, for example, PREMARIN^{®}, available from Pfizer, Inc., is supplied with a plunger type applicator for dispensing a cream into the vaginal canal; ESTRACE^{®}, available from Allergan, Inc., also is supplied with a plunger type applicator; tablets may be similarly placed deep into the vaginal canal by a plunger type applicator, *e.g*., VAGIFEM^{®}, available from Novo Nordisk Health Care AG, is supplied with an applicator to place a tablet within the vaginal canal, INTRAROSA^{™}, available from Endoceutics, Inc., is supplied with a plunger type applicator to place inserts into the vaginal canal), or insertable rings (e.g., ESTRING^{®} available from Pfizer, Inc., which is likewise inserted into the vaginal canal).

While hormonal therapies have shown positive effects, particularly in the treatment of vaginal atrophy, some women continue to experience the symptoms, and for many women, such treatment can prove to be too expensive. Hormone replacement therapy has also been reduced by contraindications such as a history of cancer and thromboembolism. Moreover, due to the nature of the condition, women may feel uncomfortable and/or embarrassed discussing the above-described symptoms and may avoid seeking a doctor's consultation. Additionally, many women stop annual visits to gynecologists, leaving their primary care family physician as the main resource, yet few primary care physicians address or treat menopausal symptoms since menopause is viewed as a "natural" process.

There are also several over-the-counter solutions that have been offered to consumers to treat various symptoms and/or conditions experienced by women. These include vaginal moisturizers (*e.g*., REPLENS^{®} Long-Lasting Moisturizer and REPLENS^{®} Moisture Restore External Comfort Gel, or HYALOGYN^{®}/HYALOFEMME^{®}, available from Fidia Farmaceutici SpA and both supplied with disposable applicators to place in the vaginal canal), lubricants for reducing discomfort during intimacy (*e.g*., REPLENS^{®} Silky Smooth Personal Lubricant, ASTROGLIDE^{®}, K-Y^{®} gels and lubricants), wipes (*e.g*., VAGISIL^{®} Anti-Itch Medicated Wipes), sprays, and washes and douches for eliminating bacteria that can cause unpleasant odors (*e.g*., SUMMER'S EVE^{®}). The REPLENS^{®} Long-Lasting Moisturizer, available from Church & Dwight Co., Inc., is provided with a plunger type applicator for depositing the moisturizer within the vaginal canal. The makers of REPLENS ^{®} have published a number of studies regarding the benefits of using the REPLENS^{®} Long-Lasting Moisturizer (*see, e.g*., https://www.womenshealthcaresolutions.com/clinical-studies/replens/).

While the aforementioned over-the-counter solutions may be useful, some may not prioritize women's intimate health or the usage experience. Many of these solutions require a separate applicator and composition, which may add complexity and may be messy to use. Some options may have hygienic drawbacks, for example, not providing an applicator and requiring the user to apply the composition with cleaning and/or bodily fluids. Many over-the-counter plunger type applicators are designed to deliver a composition deep into the vaginal canal; such applicators typically deliver large doses of composition, which may subsequently leak out of the vaginal canal. Some over-the-counter products require a user to hold an applicator in an exact orientation, in order to properly dispense the composition inside the applicator. And, some products apply composition only to the vaginal canal and not to the introitus or external vaginal tissues. These drawbacks can make for an unpleasant experience and inhibit regular adoption for consistent habit formation.

US 2008161752 A1 relates to applicators for introducing a therapeutic substance into a body cavity. The applicators have a first outer member, a dispensing end and a second end distal to the dispensing end. The applicators also have at least one aperture located in a side wall of the outer member.

US 2003088217 A1 is concerned with applicators for delivering a formulation into a user's mucosal cavity. They comprises a body having a external wall provided with perforations and internal wall spaced from said external wall to define a diffusion channel. Expulsion element forces expel a formulation out of a reservoir into the diffusion channel to deliver the formulation to the mucosal cavity through the perforations.

WO2006014572A1 discloses a delivery system comprising an applicator for dispensing a medicament to the vaginal cavity. The applicator has an elongated body having a proximal dispensing end and a distal grasping end, wherein a portion of the body forms a reservoir. There are opportunities for improvement. For example, it would be advantageous to provide applicators and methods for treating the vagina, particularly the vaginal introitus and, optionally, one or more external vaginal tissues, where the applicators have a vaginal care composition stored therein. It would also be advantageous to provide applicators that enable topical spreading of a composition onto the introitus and/or external vaginal tissues. It would be beneficial to provide applicators that deliver properly metered doses of composition. It would be further advantageous to provide an applicator that is easy to clean, while avoiding contamination of the composition within the applicator; to provide an applicator that reduces the need for a female user to touch the composition or spread it about the applicator; to provide an applicator that can be held in any orientation without leaking, dripping, or otherwise improperly dispensing the composition; to provide an applicator having a geometry and finger offset distance that enables topical spreading of the vaginal care composition without self-touch; and/or to provide reusable, multi-use applicators.

### SUMMARY

The present disclosure relates to an applicator for treating one or more vaginal tissues, comprising: a tapered, preferably rounded, insertion portion comprising one or more dispensing openings, preferably the one or more dispensing openings are free of a frangible seal; a body disposed adjacent the insertion portion comprising a reservoir in fluid communication with the one or more dispensing openings, wherein the reservoir stores greater than 10g to about 200g, preferably greater than 10g to about 100g, more preferably greater than 10g to 50g, even more preferably from about 15g to 30g of a vaginal care composition.

### BRIEF DESCRIPTION OF THE DRAWINGS

The above-mentioned and other features and advantages of the present disclosure, and the manner of attaining them, will become more apparent and the disclosure itself will be better understood by reference to the following description of non-limiting embodiments of the disclosure taken in conjunction with the accompanying drawings, wherein:
FIG. 1 is a front view of a non-limiting example of an applicator comprising an insertion portion and a body;
FIG. 2A is a cross-sectional view of a non-limiting example of an insertion portion suitable for use with the body of FIG. 1;
FIG. 2B is a bottom perspective view of the insertion portion of FIG. 2A;
FIG. 3 is a cross-sectional front view of the applicator shown in FIG. 1;
FIG. 4 is a cross-sectional front perspective view of one example of an aerosol valve suitable for use with either an insertion portion or a body;
FIG. 5 is a cut-away front perspective view of a body comprising the aerosol valve of FIG. 4;
FIG. 6A is a cross-sectional front view of a one example of a flexible umbrella valve suitable for use with a body or an insertion portion, showing the flexible umbrella valve in a closed position;
FIG. 6B is a cross-sectional front view of the flexible umbrella valve of FIG. 6A, showing the flexible umbrella valve in an open position;
FIG 6C is a cross-sectional front view of a one example of a rigid umbrella valve suitable for use with a body or an insertion portion, showing the rigid umbrella valve in an open position
FIG 6D is a cross-sectional front view of the rigid umbrella valve of FIG. 6C, showing the rigid umbrella valve in a closed position;
FIG. 7 is a cross-sectional front view of one example of a ball valve suitable for use with a body or an insertion portion, showing the ball valve in a closed position;
FIG. 8 is an exploded side perspective view of a non-limiting example of an insertion portion of an applicator or a complete applicator;
FIGS. 9A to 9J are perspective views of some non-limiting outer shapes that are suitable for use as cone-shaped insertion portions of an applicator;
FIG. 10 is a perspective front view of a non-limiting example of a body, wherein interior features are shown in dashed line for purposes of illustration;
FIG. 11 is another non-limiting example of a body; and
FIG. 12 is a cross-sectional front view of another non-limiting example of an applicator comprising an insertion portion and a body.
FIG. 13A is a cross-sectional view of the complete applicator shown in FIG. 8 in a non-dispensed position;
FIG. 13B is a cross-sectional view of the complete applicator shown in FIG. 8 in a dispensed position.
FIG. 14 is a front view of another example of an applicator not according to the claimed invention; and
FIG. 15 is a cross-sectional view of the applicator of FIG. 14.
FIG. 16 is an exploded side perspective view of a non-limiting example of an applicator comprising an insertion portion, a body, and a removable seal;
FIG. 17 is a cross-sectional view of the insertion portion shown in FIG. 16 attached to the body, wherein a portion of the body has been omitted for simplicity;
FIG. 18 is a cross-sectional view of a plurality of insertion portions (or complete applicators) of FIG. 8 shown in a nested/stacked arrangement;
FIG. 19A is a front view of a non-limiting example of another applicator;
FIG. 19B is a cross-sectional view of the applicator of FIG. 19A;
FIG. 19C is a front view of a non-limiting example of another applicator;
FIG. 19D is a cross-sectional view of the applicator of FIG. 19C.
FIG. 19E is an exploded view of the applicator of FIG. 19A.
FIG. 19F is an exploded view of the applicator of FIG. 19C.
FIG. 20A is a side view of a non-limiting example of yet another applicator;
FIG. 20B is a front view of the applicator of FIG. 20A;
FIG. 21 is a cross-sectional view of a non-limiting example of another applicator.
FIG. 22A is a front perspective view of a non-limiting example of an insertion portion comprising a thread;
FIG. 22B is a front perspective view of a non-limiting example of an insertion portion comprising a bayonet fitment;
FIG 23 is an exploded view of an insertion portion and a quick release structure.
FIG. 24A to 24C are front perspective views of a non-limiting example of an applicator.

Other features and advantages of the invention will be apparent from the following detailed description and from the claims.

### DETAILED DESCRIPTION

Various non-limiting embodiments of the present disclosure will now be described to provide an overall understanding of the principles of the function, design and use of the applicators, compositions, kits, and methods disclosed herein. One or more examples of these non-limiting embodiments are illustrated in the accompanying drawings. Those of ordinary skill in the art will understand that the embodiments and methods described herein and illustrated in the accompanying drawings are non-limiting example embodiments and that the scope of the present disclosure is defined solely by the claims. The features illustrated or described in connection with one non-limiting embodiment can be combined with the features of other non-limiting embodiments. Such modifications and variations are intended to be included within the scope of the present disclosure.

All percentages are by weight of the vaginal care composition, unless specifically stated otherwise. All ratios are weight ratios, unless specifically stated otherwise. All ranges are inclusive of narrower ranges and combinable. Delineated upper and lower range limits are interchangeable to create further ranges not explicitly delineated. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at approximately 25° C and at ambient conditions, where "ambient conditions" means conditions under about one atmosphere of pressure and at about 50% relative humidity.

The compositions of the disclosure can comprise, consist essentially of, or consist of, the components as well as optional ingredients described herein. As used herein, "consisting essentially of" means that the applicator, composition, or component may include additional ingredients or features, but only if the additional ingredients or features do not materially alter the basic and novel characteristics of the claimed applicators, compositions or methods. As used in the description and the appended claims, the singular forms "a," "an," and "the" are intended to include the plural forms as well, unless the context clearly indicates otherwise. Unless otherwise defined, all technical and scientific terms used herein have the same meaning as commonly understood by one of ordinary skill in the art to which this invention belongs.

"Attachment structure" means any structure that is configured to engage and removably couple an insertion portion and a body.

"Body" means a structure, device or article of manufacture having a grippable portion and which is either (i) removably attachable to an insertion portion, or (ii) formed with, attached to, engaged with, and/or connected to an insertion portion. A body may or may not comprise a reservoir.

"Estrogen agent" means any natural or synthetic estrogen hormone (*e.g*., estrone, estradiol and estriol), metabolites thereof, esters thereof, analogues thereof; phytoestrogens (*e.g*., isoflavones, coumestans, prenylflavonoids); estrogen precursors (*e.g*., dehydroepiandrosterone); and/or any compound which binds to an estrogen receptor or which otherwise exhibits at least mild or weak estrogen-like effects, including selective estrogen receptor modulators ("SERM") such as, *e.g*., afimoxifene (4-hydroxytamoxifen), arzoxifene, bazedoxifene, clomifene, femarelle (DT56a), lasofoxifene, ormeloxifene, raloxifene, tamoxifen, toremifene, mifepristone (RU486), VA2914, ulipristal, Proellex, Asoprisnil, and CDB-4124.

"External vaginal tissue" means one or more of the external female genital organs that are visible and bounded longitudinally by the mons pubis and anus and bounded laterally by the genitocrural folds, including but not limited to the vulvar vestibule, labia majora, labia minora, external urogenital tract, urethral orifice, clitoris, and vulvar skin.

"Fingertip offset distance" means the distance between (1) the hyponychium of the fingertip closest to the tip of an applicator while grasping the applicator during use, and (2) the tip of such applicator.

"Fluid communication" means that a flowable composition is movable between one structure or feature and a second structure or feature, either continuously or intermittently. Two structures are considered to be in fluid communication even though there are intermediate structures (*e.g*., a valve, pump, etc.) disposed between the two structures.

"Grippable portion" means a portion having an outer shape and size which may be grasped by two or more fingers or fingertips of a human hand to manipulate the applicator in use.

"Medical grade" means a material that passes either i) one or more of the United States Pharmacopeia and National Formulary (USP-NF) Class IV, V, and Class VI designations, or ii) ISO 10993 testing for one or more of cytotoxicity (ISO 10993-5), 7-day implant (ISO 10993-6), skin irritation (ISO 10993-10), and skin sensitization (ISO 10993-10).

"Micro-texture" means a texture or surface finish having a surface texture, Sq, from about 3 µm to about 30 µm, or from about 3.3 µm to about 20 µm, or from about 3.5 µm to about 10 µm as measured by the Surface Texture Procedure below.

"One-way valve" means a valve that allows a fluid, for example, the vaginal care composition, to flow in one direction and to reduce or prevent fluid flow in the reverse direction.

"Peri-menopausal" woman is one who, in the absence of hormone replacement therapy or other medication, would experience a change in her intermenstrual cycle interval and have associated symptoms of estrogen deficiency, such as vasomotor flushes, vaginal dryness, and/or worsening premenstrual syndrome. Also, included are women who in the absence of hormone replacement therapy or other medication would experience less than 12 months of amenorrhea.

"Pharmacologically effective amount", "therapeutically effective amount", or simply "effective amount" means the amount of a composition, or ingredient thereof, effective to produce the intended pharmacological, therapeutic, or preventive result.

"Post-menopausal" woman is one who in the absence of hormone replacement therapy or other medication would experience at least 12 months of amenorrhea.

"Progesterone agent" means any natural or synthetic progesterone hormone, metabolites thereof, analogues thereof, progesterone precursors, and/or any compound which binds to a progesterone receptor or which otherwise exhibits at least mild or weak progesterone-like effects, including selective progesterone receptor modulators ("SPRM") such as, for example, telapristone.

"Reservoir" means the space or volume which contains a vaginal care composition.

"Rotational symmetry" means the applicator, or a portion thereof such as the insertion portion or the grippable portion or the body, has an overall shape that looks the same (except with respect to its surface features) through some rotation (*e.g*., 45°, 90°, 135°, 180°, 225°, 270°, 315°, or 360°) about its longitudinal axis. For example, an applicator that has an overall shape that looks the same through a rotation of 45° is considered rotationally symmetrical through that 45° rotation. Likewise, an applicator that has an overall shape that looks the same through one full rotation is considered rotationally symmetrical through 360°. The reference to rotational symmetry herein, unless stated otherwise, ignores surface features such as print, coloring, coatings, text, graphics, dosing indicators, insertion indicators, surface textures, and surface finishes.

"Single-use applicator" means an applicator to be used once and disposed thereafter.

"Smooth" means a surface having a surface texture (Sq) less than about 30 µm, or less than about 10 µm, or less than about 3 µm as measured by the Surface Texture Procedure below. Smooth surfaces include surfaces having a micro-texture.

"Substantially free" means a component or material is present in amount less than 0.1%, 0.05%, 0.025%, 0.01%, or 0.001% by weight of the vaginal care composition.

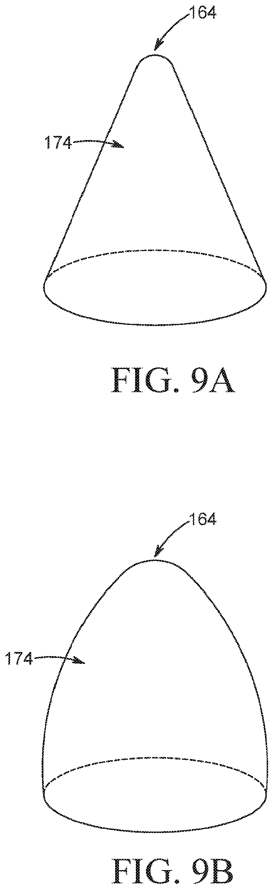

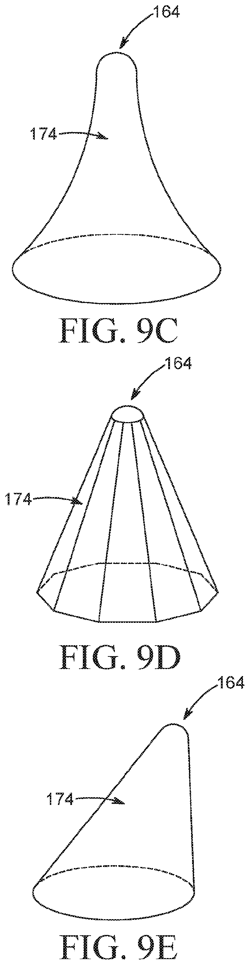

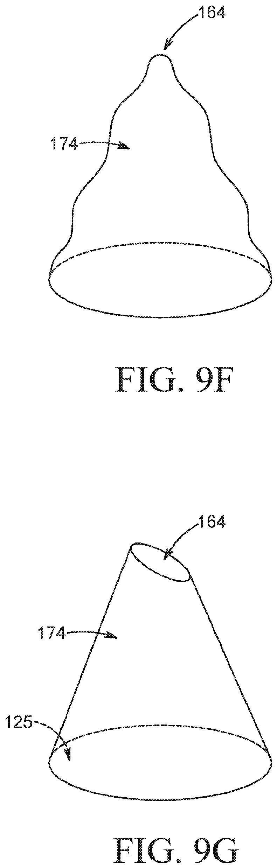

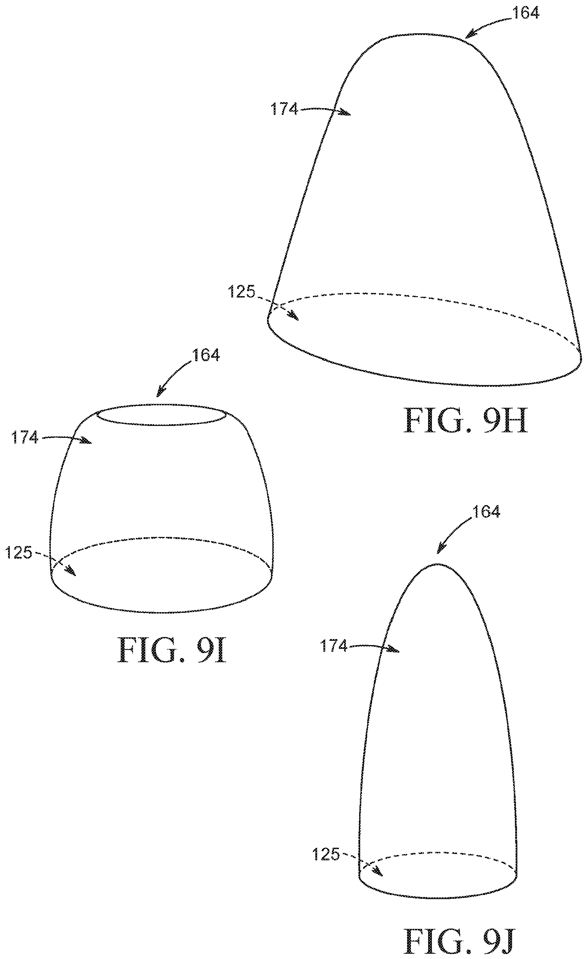

"Taper" means to become smaller toward one end. The taper may be gradual, substantial, intermittent, continuous, and combinations thereof. For example, an insertion portion is considered to taper from the maximum width to the tip merely if the bulk cross-sectional area (*e.g*., inclusive of both solid cross-sectional area and void cross-sectional area) at the tip is less than the bulk cross-sectional area at the maximum width, even though, for example, the bulk cross-sectional area may intermittently increase or remain constant between the maximum width and the tip. Some non-limiting examples of tapered insertion portions are shown in FIGS. 9A to 9J. A continuous taper is a taper wherein the bulk cross-sectional area of the applicator decreases along at least 80%, 90%, 95%, or 100% of a traversal from a first point on the longitudinal axis to a second point on the longitudinal axis of the applicator (*e.g*., from maximum width (Wₘₐₓ) of an insertion portion to its tip). Some non-limiting examples of a continuous taper are shown in FIGS. 9A to 9J.

"Vaginal care composition" means any composition that is suitable for application to the vaginal introitus and/or one or more external vaginal tissues.

"Vaginal introitus" or "introitus" means the opening of the vaginal canal adjacent to the vulvar vestibule.

### A. Applicators for Use in Treating the Vaginal Introitus and, Optionally, One or More External Vaginal Tissues

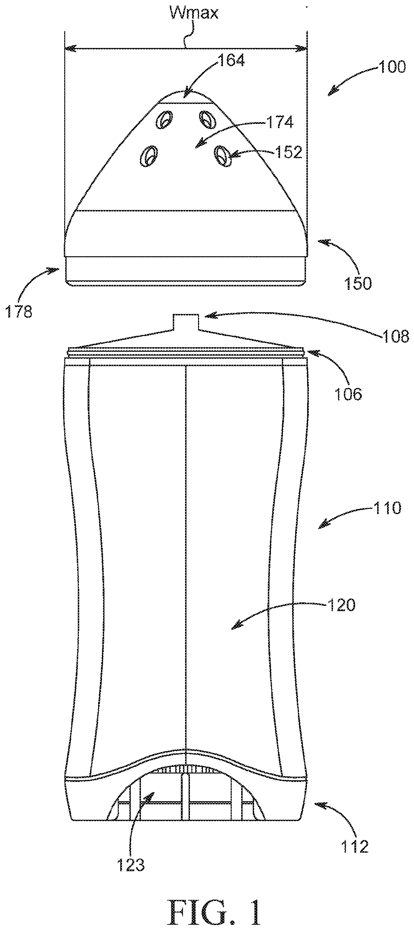

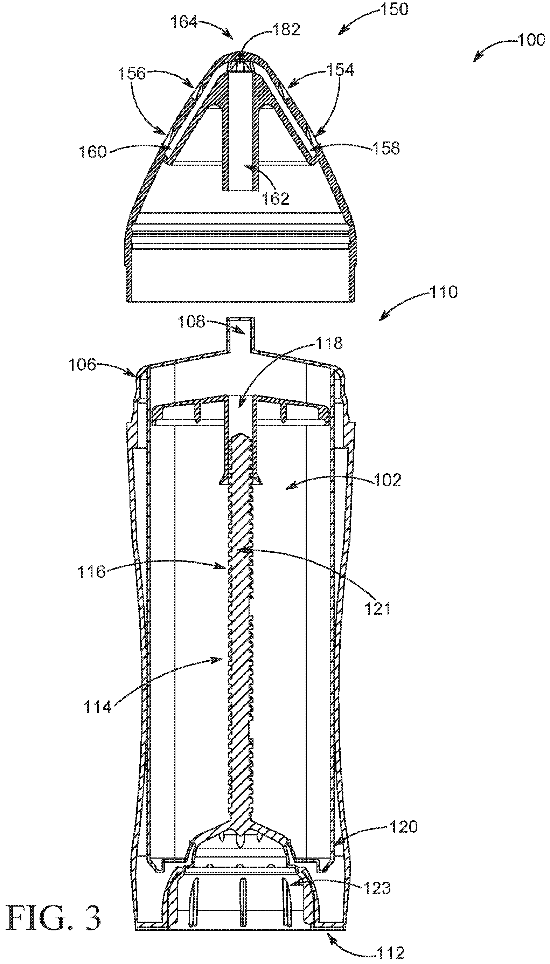

Referring now to FIGS. 1 to 3, a non-limiting example of an applicator is described. As shown in FIG. 1, the applicator 100 comprises a body 110 and an insertion portion 150. The body comprises a reservoir 102 (*see, e.g*., FIG. 3) having a vaginal care composition disposed therein. The applicator 100 may provide a better user experience by eliminating the need for a separate dispenser apart from the applicator (*e.g.,* a hand pump) to dispense a vaginal care composition onto the outer surface 174 of the insertion portion 150.

The insertion portion 150, which may be permanently or removably attached to the body 110, comprises one or more dispensing openings 152 and a tip 164, as shown in FIG. 1. A dosage of the vaginal care composition is dispensed through the dispensing openings 152 and onto the outer surface 174 of the insertion portion 150. The insertion portion 150 tapers from its proximal end (*e.g.,* 178 shown in FIG. 1). A female user may administer the dosage of the vaginal composition to her vaginal introitus and, optionally, one or more external vaginal tissues by grasping and manipulating the applicator 100. The dosage of the vaginal care composition may be dispensed prior to, during, and/or after contacting the vaginal tissues with the applicator.

The applicator is a multi-use applicator and the reservoir stores greater than 10g to about 200g, or greater than 10g to about 100g, or greater than 10g to 50g, or from about 15g to 30g of the vaginal care composition. The reservoir 102 may hold a sufficient quantity for multiple doses/uses over several days or weeks without the need to refill. The dosage of the vaginal care composition dispensed onto the outer surface of the insertion portion may be from about 0.1g to about 2g, from about 0.2g to about 1.2g, or from about 0.3g to about 1.1g per usage of the applicator. Stated another way, the aforementioned dosages are dispensed onto the lateral outer surface 174 and applied for each treatment by the female user. In some embodiments, the reservoir is not refilled between uses. In some examples, the dose is about 100%, 50%, 25%, 10%, 5%, 3%, 2%, 1% or 0.5% by weight of the vaginal care composition originally stored in the reservoir.

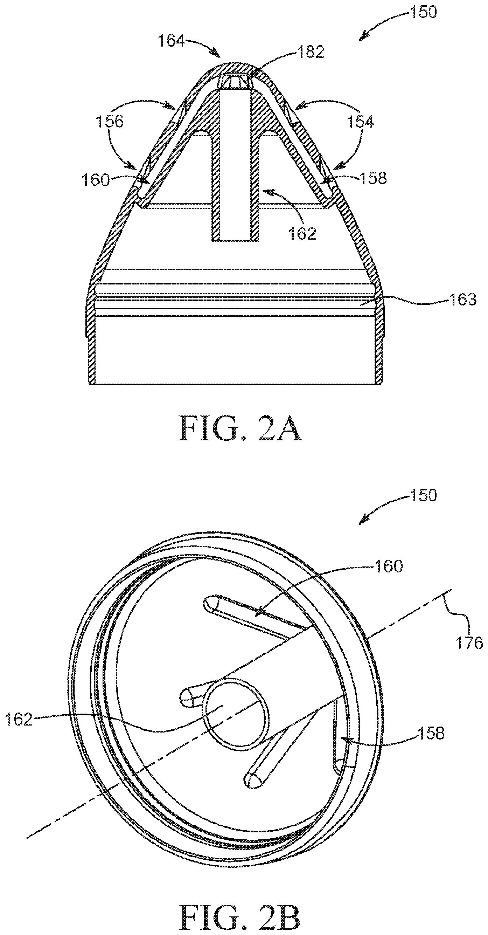

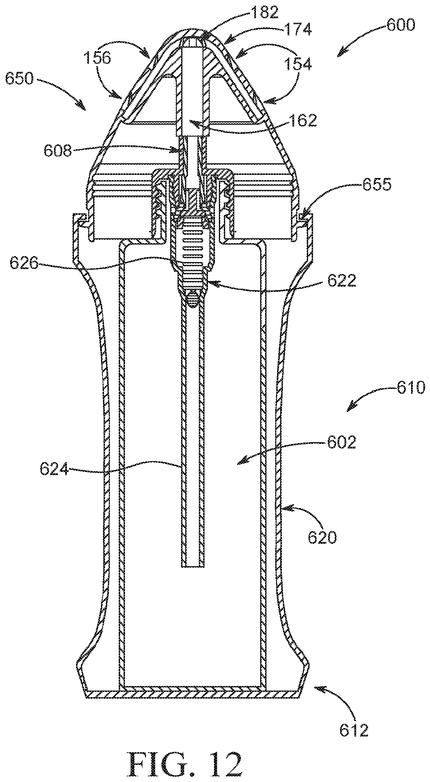

Referring to FIG. 12, a multi-use applicator is illustrated. Applicator 600 comprises an insertion portion 650 and a body 610 comprising a shell 620, a pump 622 with a dip tube 624 extending into reservoir 602. The reservoir 602 comprises a vaginal care composition. The insertion portion 650 may be similarly configured as previously described in connection with insertion portion 150 of FIGS. 2A and 2B, except the insertion portion 650 may further comprise a radially extending lip 655 that engages the body 610 to capture the insertion portion 650 within the body 610. The pump 622 is actuated by depressing the insertion portion 650, which is biased by spring 626 of pump 622. Actuation of the pump 622 in turn dispenses a dosage of the vaginal care composition from the reservoir 602 to the insertion portion 650 and out of the dispensing openings 154 and 156 and onto the outer surface 174 of the insertion portion 650.

Referring to Figs. 1 and 3, the insertion portion 150 of the applicator 100 comprises a plurality of dispensing openings 152, which may reduce or eliminate the need for a female user to touch the vaginal care composition to spread it around the insertion portion prior to application to the vaginal tissue. The body 110 may comprise a reservoir 102 disposed within a sleeve 120, a male snap-fit 106 in the form of a radially extending ring, and a proximal end 112, which may also be the grippable portion. As described in more detail below, a vaginal care composition is stored within the reservoir 102 of the applicator 100. The reservoir 102 is in fluid communication with the dispensing openings 154,156, also described in greater detail below. The transition of the outer surface 174 of the insertion portion 150 and the sleeve 120 of the body 110 may be generally smooth and free of sharp edges or crevices. This prevents the vaginal care composition from gathering and hardening if the reservoir allows for multiple uses of the insertion portion. Likewise, preventing the collection of the vaginal care composition promotes improved hygiene and comfort in use. In addition, a lack of sharp edges will avoid irritating, scraping, or scratching the delicate vaginal tissues during use.

The applicator 100 may have an overall length from about 25mm to about 175mm, or from about 35mm to about 150mm, or from about 45mm to about 125mm. The size of the applicator and the inclusion of a reservoir within the applicator 100 improves portability, while reducing the amount of packaging required. The integrated design also provides the benefit of simplicity, without concern for separate parts or the chance of inadvertently misplacing a piece of a multi-part kit. In some examples, the applicator 100 is a multi-use applicator.

Referring now to FIGS. 2A, 2B, a non-limiting example of an insertion portion 150, which is suitable for use with the body of FIG. 1, is depicted for topically applying a vaginal care composition to the vaginal introitus and/or one or more external vaginal tissues. The insertion portion 150 may be removably attached to the body 110 (where the insertion portion can be removed following use, disposed of separately, and/or a new or fresh insertion portion may be attached to the body), integrally formed with the body, or otherwise attached in a manner not easily removed by a consumer. The lateral, outer surface of an insertion portion may be smooth and free of sharp edges and crevices, although a smooth surface may still have a micro-texture, which may be provided by any number of methods known in the art, e.g., sand blasting injection molds. A smooth surface can avoid irritation of the vaginal tissues during manipulation of the applicator to administer the vaginal care composition to the vaginal tissues of interest. Preferably, the micro-texture, while visible to a user, is not felt by a female user in use when the insertion portion is inserted into the vaginal introitus or rubbed against the external vaginal tissues of interest. In addition, a micro-texture can reduce soiling and/or tackiness of the insertion portion. The micro-texture may cover 50%, 60%, 70%, 80%, 90%, or 100% of the insertion portion.

The dimensions and shape of an insertion portion may facilitate use at the vaginal introitus and, optionally, external vaginal tissues. While there may be considerable variability in vaginal shape, axis, and dimension from woman to woman, the dimensions and shape of the insertion portion may be designed to accommodate the vaginal introitus, taking into consideration a wide range of anatomical measurements, while also self-limiting the insertion depth of the applicator. In a study by Luo *et al.* entitled, "Quantitative analyses of variability in normal vaginal shape and dimension on MR images" ("Luo *et al.*")*,* magnetic resonance imaging (MRI) was used to take a series of measurements in order to quantify variability in vaginal dimensions for a group of women age 28 years to 70 years. Luo *et al.* recognized that the vagina has three regions: a lower region (distal half of an anterior vaginal wall (AVW)), a middle region (proximal half of the AVW), and an upper region (cervical portion axis). Among its measurements, Luo *et al.* also assessed vaginal widths along a vaginal length. Luo *et al.* notes that the vaginal width is generally at its largest in an upper region of the vagina but generally decreases toward a lower region, such that the width is at its narrowest at the vaginal introitus. Lou *et al.* noted that the vaginal threshold (introitus) had a minimum dimension of 9mm, maximum of 31mm and mean of 17mm, with a standard deviation of 5mm, which may represent a dimensional range for peri-menopausal and post-menopausal woman. In some examples, the maximum width Wₘₐₓ of the insertion portion can be from about 20mm to about 60mm; from about 25mm to about 55mm, from about 30mm to about 50 mm or any value from about 20mm to about 60mm, or any range formed by any of the preceding values.

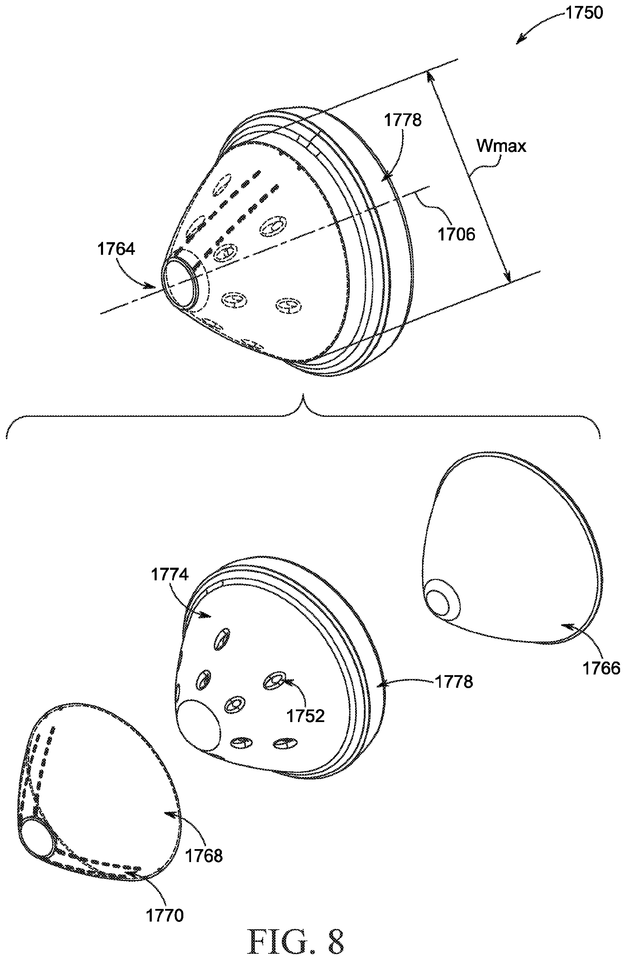

In some examples, it may be desirable for the applicator (and in some embodiments, the insertion portion in particular) to be dimensioned to prevent over-insertion of the insertion portion into the vaginal canal. In some examples, the width of the applicator at a longitudinal distance of 25mm from the tip of the insertion portion is from about 15mm to 45mm, or from about 20mm to about 40mm, or from about 25mm to about 35mm. The longitudinal distance is the distance measured along the longitudinal axis or centerline (e.g., 1706 in FIG. 8) passing through the tip of the insertion portion. In some embodiments, the insertion portion 150 may have an overall length from about 25mm to about 65mm, or from about 25mm to about 55mm, or from about 25mm to about 50mm.

While a variety of overall shapes may be provided for the insertion portions shown and/or described herein, in some examples the insertion portion may be further described as rotationally symmetrical through 360° of angular rotation about the longitudinal axis 1706 thereof, although it is recognized that bodies of other applicators may be rotationally symmetrical through less than 360° *(e.g.,* 45°, 90°, 135°, 180°, 225°, 270°, 315°, or 360°). It is believed this geometric shape is desirable for topical application of a spreadable vaginal care composition to the vaginal introitus and, optionally, one or more external vaginal tissues. This shape may also be particularly useful in the case of women suffering from vaginal atrophy, given the variability in shape and size of this vaginal tissue between users as well as the sensitivity of these tissues. It is also believed that this shape can facilitate grasping and manipulating the applicator by a user to apply the vaginal care composition easily to the introitus and external vaginal tissues while in a variety of positions (*e.g*., seated, standing, or laying down) and without viewing the tissues of interest during use.

While the insertion portions may be provided in a variety of shapes, the insertion portion tapers from its proximal end (*e.g.,* 1778 shown in FIG. 8) to its tip. In some embodiments, the insertion portion is cone-shaped. Referring now to FIG. 9A-9J, some non-limiting cone-shaped geometries are shown such as a right circular cone, a multi-faceted cone, an oblique cone shown, and a contoured cone shown. In some examples, a side profile of a lateral surface 174 of the cone shaped insertion portion may be at least partially convex, at least partially concave, or at least partially straight. The tip 164 of the insertion portion 150 may have any shape, including but not limited to rounded, concave, convex, flat, indented, angled, or pointed shape.

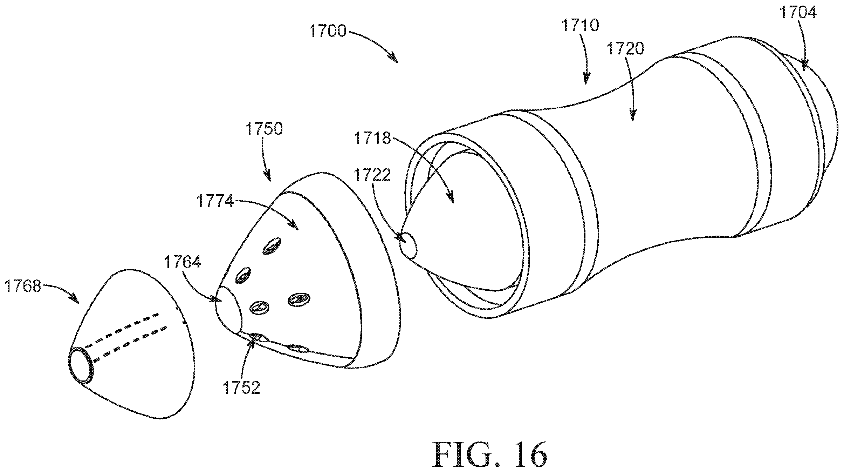

Referring to FIG.8, a non-limiting example of an insertion portion (similar to the insertion portion shown in FIG. 16) is shown in both assembled and exploded views. The insertion portion 1750 includes dispensing openings 1752, a tip 1764, removable seal 1768, perforations 1770 for tearing the seal for removal, lateral outer surface 1774, a proximal end 1778, and a plunger 1766. The removable seal 1768 may cover the dispensing openings 1752 or the entire outer surface of the insertion portion. The seal may be formed from a flexible film or provided as a rigid cap (with or without perforations or a tear strip) or other sealing means.

In general, the lateral outer surface 1774 of the insertion portion 1750 may be smooth and free of sharp edges and crevices, although a smooth surface may still have a micro-texture which may be provided, for example, by sand blasting injection molds for the body. As with other embodiments disclosed herein, a smooth surface can avoid irritation of the vaginal tissues during manipulation of the applicator to administer the vaginal care composition to the vaginal tissues of interest. The micro-texture, while visible to a user, is not felt by a female user in use when the insertion portion 1750 is inserted into the vaginal introitus or rubbed against the external vaginal tissues of interest. In addition, a micro-texture can reduce soiling and/or tackiness of the insertion portion. The micro-texture may cover 50%, 60%, 70%, 80%, 90%, or 100% of the insertion portion 1750. Alternatively, as discussed below, the illustrations in FIG. 8 may represent a complete applicator, such as a single-use applicator (rather than an insertion portion of a larger applicator).

### Removable Insertion Portion

Applicators that have removably attached insertion portions may provide a more hygienic use experience by allowing consumers to remove and clean the insertion portion or use a new insertion portion for each application.

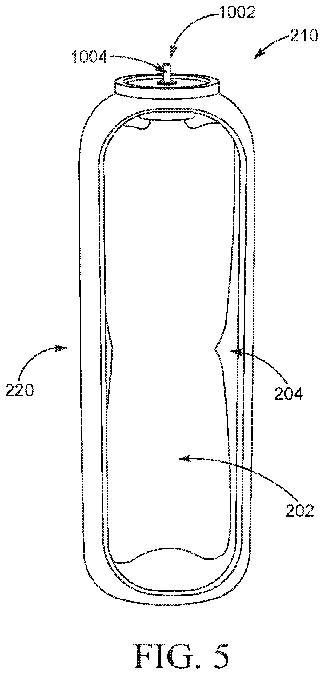

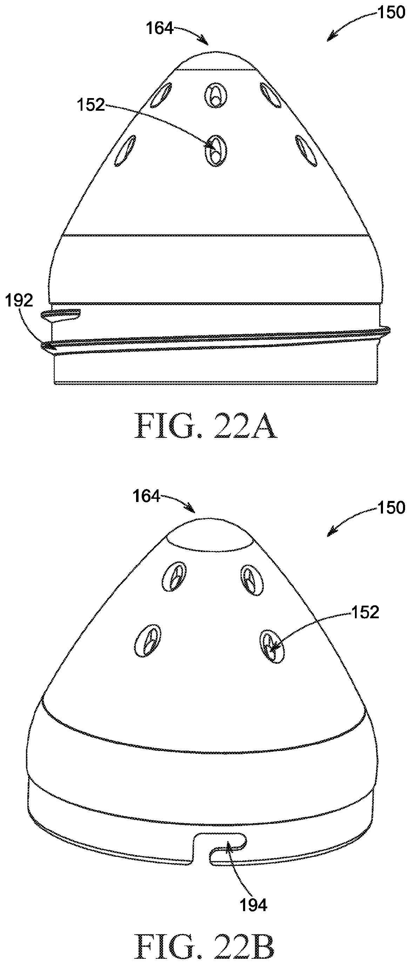

If removable, the insertion portion may comprise any number of different means for attaching it to the body. FIGS. 22A and 22B, for example, depict various attachment structures disposed on an insertion portion. It will be appreciated, though, that the same attachment structures may alternatively be disposed on a body. At least one of the insertion portion or its corresponding body may comprise either a recess or a protrusion that engage one another to removably attach the insertion portion to the body. In some examples, the recess and protrusion may be provided in the form of male and female threads, a bayonet fitment, or a groove and ring. Alternatively, an insertion portion and body may removably engage by means of a friction or interference fit or magnets or other means, such as Velcro. For example, the insertion portion 150 may be removably attached by way of a friction fit or snap fit between a stem 1004 of a body 210 (shown in FIG. 5) and a conduit 162 (shown in FIGs. 2A and 2B). The conduit 162 may be in the form of a hollow cylinder (although other shapes may be provided) in the insertion portion 150. The insertion portion 150 may be removably attached using a female snap-fit connection 163 of the insertion portion 150, which engages a male snap-fit 106 of the body 110 (shown in FIGs. 1 and 2A, respectively).

With regard to a press fit, also referred to as an interference fit, the insertion portion 150 may comprise a recess in the form of an annular channel (not shown) that removably engages a corresponding ring (not shown) on the mating body so that the insertion portion 150 may be removably attached to the body. Alternatively, or in addition to the interference fit described above, the insertion portion may comprise a conduit 162, as shown in FIG. 2B, and the conduit 162 may have a tapered inner surface (not shown) that engages a corresponding tapered outer surface of an upstanding channel (for *e.g*., 108 shown in FIG. 1) of the mating body. The tapered inner surface of the insertion portion and tapered outer surface of the channel of the body are configured to provide a separable friction fit sufficient to retain the insertion portion on the body during use, but not so tightly that the insertion portion cannot be removed by a female user following treatment.

Referring to FIG. 22A, an insertion portion 150 is illustrated that comprises a protrusion (or first attachment structure) in the form of a male thread 192, also sometimes referred to as a helix or a twist connection. The corresponding body has a mating second attachment structure in the form of a female thread (not shown) that engages the male thread 192 in a threaded engagement. Referring to FIG. 22B, an alternate embodiment of insertion portion 150 is illustrated that comprises a recess (or first attachment structure) in the form of a bayonet slot 194. The bayonet slot 194, as shown in FIG. 22B, comprises an inter-lockable cutout or hole that engages a mating second attachment structure in the form of a protrusion (not shown) on the body. The bayonet slot, for example, is L-shaped.

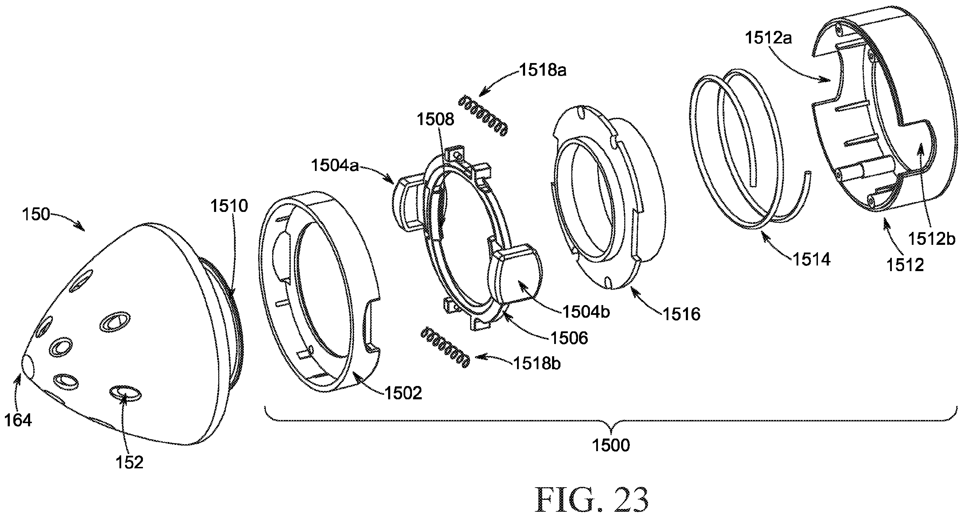

Referring now to FIG. 23, another embodiment of the insertion portion 150 is illustrated that engages a snap-on quick release structure 1500 of a body (the remainder of the body is not shown for simplicity). The insertion portion 150 is fitted to a ring 1502. The ring 1502 is attached to or integrally formed with a body. The ring 1502 comprises a set of radially displaceable engagement members 1504a and 1504b (although only one may be provided) that protrude outside the surface of the ring 1502. The engagement members 1504a and 1504b are extensions of a collar 1506 that is disposed within the ring 1502. As shown in FIG. 23, a wedge portion 1508 of the collar 1506 is engaged to an extended portion 1510 of the insertion portion 150 to removably position the insertion portion 150 in place. In other words, the wedge portion 1508 of the collar 1506 buttons out when the insertion portion 150 is engaged and connected to the collar 1506, which allows a snap-on fit for the insertion portion 150.

When a user desires to detach the insertion portion 150 from the ring 1502, the user presses the engagement members 1504a and 1504b radially from both sides, which disengages the wedge portion 1508 from the extended portion 1510 of the insertion portion 150. In other words, the wedge portion 1508 buttons into the collar 1506 when a pressure is inwardly applied on the engagement members 1504a and 1504b, which allows the insertion portion 150 to be quickly released from the engaged position.

The snap-on quick release structure 1500 further comprises a cup 1512, a platform 1516 longitudinally biased by a first spring 1514, and the collar 1506. The cup 1512 is configured to receive and position the platform 1516, the first spring 1514, and the collar 1506. The cup 1512 has cut portions 1512a and 1512b to allow the engagement members 1504a and 1504b to extend beyond the surface of the cup 1512 so that the user can press the engagement members 1504a and 1504b. The platform 1516 is configured to be biased by the first spring 1514 to allow the platform 1516 to be retractably positioned within the cup 1512, and the platform 1516 houses the annular portion of the collar 1506. The collar 1506 comprises the engagement members 1504a and 1504b, where the attachment structure further comprises one or more second springs 1518a and 1518b that radially bias the pair of engagement members 1504a and 1504b, and the engagement members 1504a and 1504b are slideably received by the platform 1516. The snap-on quick release structure 1500 further comprises ring 1502, wherein the collar 1506 is disposed between the ring 1502 and the platform 1516 and is retained or captured by the ring 1502 and the platform 1516.

In some instances, a plurality of removable insertion portions may be provided as part of a kit, wherein two or more of the insertion portions have different sizes (e.g., different Wₘₐₓ or length), shapes, and/or are formed from different materials (or contain different vaginal care compositions if the insertion portion comprises a reservoir). In some examples, a plurality of kits may be offered for sale to female consumers as part of a product or brand line-up, wherein each of the plurality of kits comprises a plurality of insertion portions having a different size and/or shape from another kit.

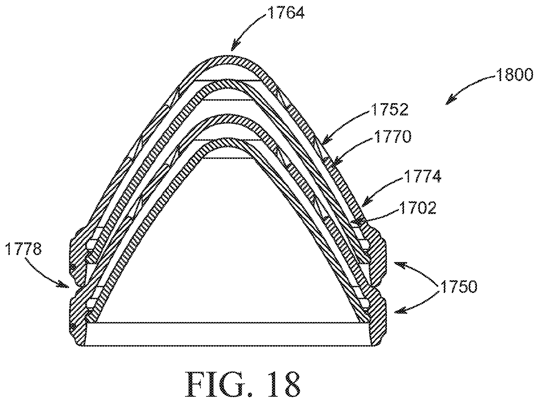

Referring now to FIG. 18, a cross-sectional view of a plurality of insertion portions 1750 (or complete applicators) are depicted in a nested/stacked arrangement. The plurality of insertion portions 1750 may be provided as a kit having a small overall volume and therefore less packaging. In use, a female user removes an insertion portion from the kit and attaches the insertion portion to a body and dispenses a dosage of the vaginal care composition onto the outer surface of the insertion portion. The female user then may apply the vaginal care composition to the vaginal tissues of interest by swirling, pivoting, rotating, revolving, or gliding the insertion portion over/on the vaginal tissues of interest. Thereafter, the female may discard/dispose of the used insertion portion. The female user repeats the steps of grasping the applicator, dispensing a dosage of the vaginal composition from the reservoir, and administering at least a portion of the dosage to her vaginal introitus, on a different day, using a different insertion portion of the kit 1800. While the kit 1800 has been illustrated and described with respect to insertion portions 1750, it will be appreciated that other embodiments of an insertion portion may be provided as kit in a nested arrangement or otherwise.

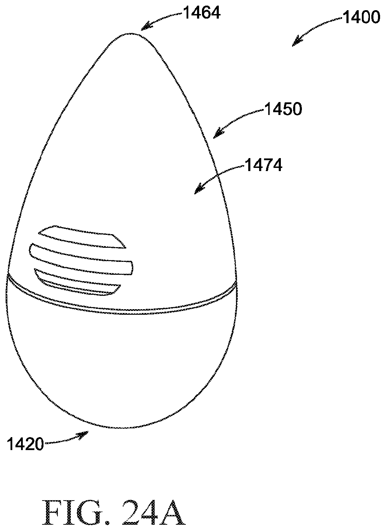

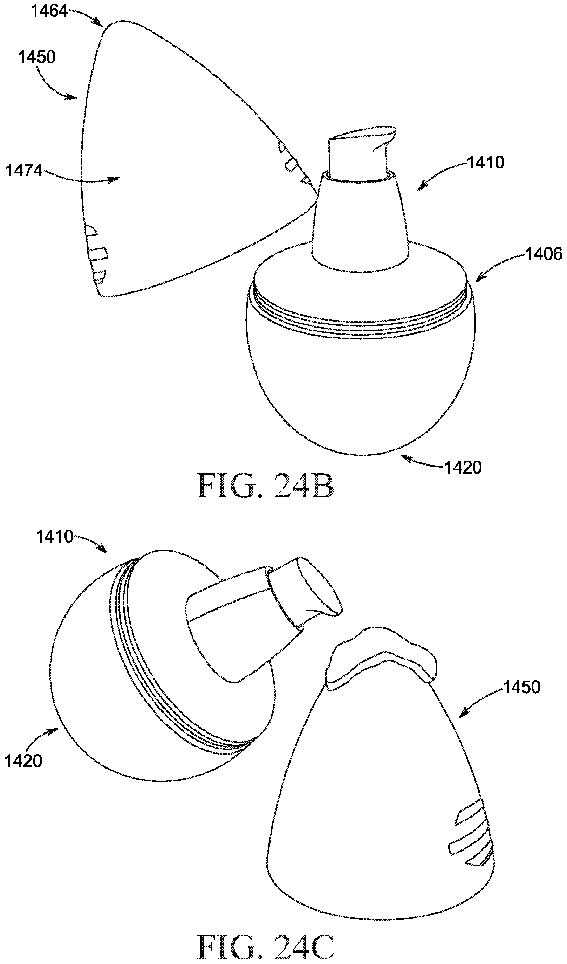

Referring now to FIGS. 24A to 24C, an applicator 1400 is depicted. The applicator 1400 comprises an insertion portion 1450, a body 1410, an attachment structure 1406 (in the form of threads, but other attachment structures may be provided), and a proximal end 1420 opposite the attachment structure 1406. The insertion portion 1450 is devoid of dispensing openings through its outer surface 1474. As shown in FIGS. 24A-24C, the body 1410 may further include a reservoir (not shown), in which a vaginal care composition is stored. A pump dispenser is provided, which is covered by the insertion portion when the insertion portion is removably attached to the body. In some embodiments, a kit comprises a plurality of insertion portions 1450, although in other embodiments, just one or a single insertion portion that is reusable may be provided. In some examples, the vaginal care composition is applied to the tip or to the outer surface of the insertion portion 1450 to be administered to the vaginal introitus and/or one or more the external vaginal tissues.

The method of using the applicator 1400 (not claimed) comprises a female user suffering from one or more of vaginal dryness, vaginal irritation, vaginal itch, vaginal infection, vaginal odor, vaginal chafing, and/or vaginal atrophy, grasping the applicator. The female user removes the insertion portion 1450 from the applicator 1400. The female user dispenses a dosage of a vaginal care composition onto the tip 1464 or to the outer surface 1474 of the insertion portion 1450 from the pump dispenser or, for example, other dispensing means described herein (e.g., by squeezing, pinching, grasping or manipulating the body). For example, instead of a pump dispenser, the body may be formed at least, in part, by a first film and a second film that are sealed about at least a portion of their peripheries, where the female user dispenses the dosage from a reservoir within the body. The female user may either (i) re-attach the insertion portion to the body and then apply the dosage of the vaginal care composition to her introitus and/or one or more external vaginal care tissues, or (ii) grasp and manipulate the insertion portion 1450 (apart from the body 1410) to apply the dosage of the vaginal care composition to the vaginal tissues of interest. In an embodiment, the method further comprises the female user removing the insertion portion 1450 from the applicator 1400. The foregoing method may be altered as desired.

After usage, the insertion portion 1450 may be rinsed (either separately from or attached to the body 1410). Alternatively, in another embodiment, the female user removably attaches a new insertion portion 1450 to the applicator 1400 by engaging the attachment structure 1406. The new insertion portion is available from a kit that includes multiple insertion portions and the removed insertion portions are disposed after use. Then, the female user dispenses a vaginal care composition onto an outer surface, for example, the tip 1464, of the new insertion portion, and administers at least a portion of the vaginal care composition to her vaginal introitus. Finally, the female user removes the new insertion portion from the applicator 1400.

### Multiple Dispensing Openings

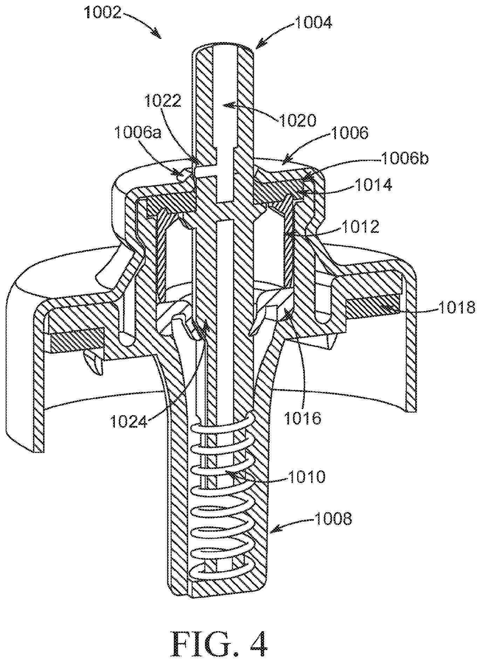

Visible in FIGS. 2A and 2B, an insertion portion 150 may comprise a plurality of dispensing openings 154 and 156 in fluid communication with channels 158 and 160, respectively and downwardly extending channel or conduit 162 having an opening at one end. FIGS. 2A and 2B show a first plurality of dispensing openings 154, a second plurality of dispensing openings 156, a first channel 158, a second channel 160, a conduit 162, which may be in the form of a hollow cylinder (although other shapes may be provided), product chamber 182, and a tip 164. While a plurality of dispensing openings is shown in FIG. 2A, one opening (*e.g*., at the tip 164) or a different arrangement of dispensing openings may be provided. During use, a dosage of the vaginal care composition flows from the reservoir 102 through reservoir channel 108 (as shown in FIG. 3) or stem 1004 (as shown in FIGS. 4-5), into the conduit 162 and then into the first and second channels (158 and 160, respectively) and, optionally, out of the dispensing openings associated with each of the first and second channels. Additional channels may be provided in fluid communication with the conduit 162 (shown in FIG. 2B), each of these additional channels having one or more dispensing openings associated therewith that are disposed in the outer surface 174 of the insertion portion 150.

The first plurality of dispensing openings 154 is in fluid communication with a first channel 158 and the second plurality of dispensing openings 156 are in fluid communication with a second channel 160. The first channel 158 and the second channel 160 are in fluid communication with the conduit 162, which is turn in fluid communication with the reservoir channel 108 and the reservoir 102 (as shown in FIG. 3). There may be a valve or other structures in between the channels and the reservoir. The first and second plurality of dispensing openings 154, 156 are also considered to be in fluid communication with the reservoir 102 when a removable insertion portion is attached to the body. While a first and second plurality of dispensing openings are discussed above for purposes of simplicity with respect to the cross-sectional view of FIG. 2A, it will be appreciated that more sets of dispensing openings and channels may be provided. Other arrangements are possible. For example, all the dispensing openings might communicate with a single channel, passage, or annulus.

The conduit 162 extends longitudinally downward or away from the tip 164 and engages a reservoir channel 108 of the body 110, as shown in FIG. 3, or a stem 1004 of the body 210, as shown in FIG. 5. In some examples, the one or more dispensing openings may include a plurality of dispensing openings, or from about 2 to about 50 dispensing openings or from about 3 to about 45 dispensing openings, or from about 4 to 40 dispensing openings. Each of the dispensing openings may have an opening dimension from about 0.1mm to about 10mm, or from about 0.25mm to about 8mm, or from about 0.5mm to about 5mm. In the case of circular shaped dispensing openings, the dimension may be the inside diameter of the circle. The openings may be sized and arranged to facilitate dispensing of an appropriate dosage (*e.g*., 0.1g to 2g) of the vaginal care composition. The opening(s) may each have an opening area of from about 0.008mm² to about 80mm², or from about 0.05mm² to about 50mm², or from about 0.2mm² to about 20mm².

In some examples, the dispensing openings may be arranged in one or more circumferential rows, circumferential columns, or a combination thereof about the insertion portion 150, although other patterns may be provided. For example, the dispensing openings might be arranged in a spiral pattern, random pattern, or other geometric pattern. Distributing the dispensing openings about the outer surface 174 of the insertion portion distributes the dosage of the vaginal care composition about the outer surface 174 of the insertion portion 150 and may minimize the need for the female user to touch the vaginal care composition. Distribution of the vaginal care composition about the insertion portion in turn facilitates ease of use of the applicator by a female user, as the applicator may be used in any orientation to apply the vaginal care composition to the introitus and optionally one or more external vaginal tissues of interest. The dispensing openings may be provided in a variety of shapes and sizes. The dispensing openings may be circular, oval, annular, square, rectangular, diamond shaped, or other geometric shapes.

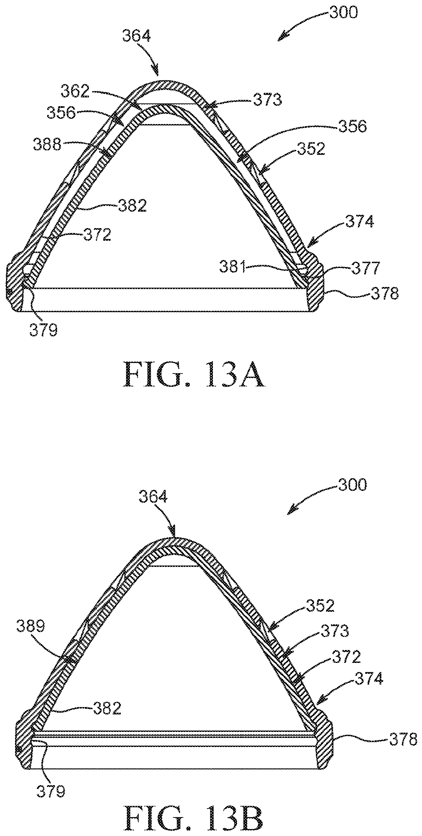

In the example depicted in FIG. 13A, the reservoir 356 (which comprises the vaginal care composition) is defined by the upper surface 362 of the plunger 388 and the inner surface 372 of the wall 373. In some examples, the plunger 388 may be rigid and provided in a cone shape. The plunger 388 may further comprise a lip 377 located on the outer surface 362 of the plunger 388 that engages a first recess 379 of the inner surface 372 of the wall 373 prior to movement of the plunger 388.

Referring to FIG. 13B, the plunger 388 is shown displaced from its position in FIG. 13A. When the plunger 388 is moved longitudinally toward the tip by applying finger pressure to the inner surface 382 of the plunger 388, the lip 377 may engage a second recess 381 (as shown in FIG. 13A) of the inner surface 372 of the wall 373. This movement decreases the volume of the reservoir thereby expelling a dosage of the vaginal care composition through the dispensing openings 352 and onto the outer surface 374 of the wall 373.

In another example, the plunger 388 may be fixedly attached to or integrally formed with the wall 373 at the proximal end 378 of the applicator. At least a portion of the plunger 388 may be formed from a flexible or stretchable material, such as an elastomeric film, that may be displaced toward the tip 364 by application of finger pressure to the inner surface 382 of the plunger 388.

The wall 373 of the applicator may be shaped and sized to accommodate the anatomical geometry of the vaginal introitus and for applying the vaginal care composition thereto. The wall 373 may have a size and/or outer shape the same as or similar to any of the insertion portions described herein. Other arrangements of the dispensing openings 352 may be provided.

In some examples, the body 1810 may be formed at least in part by a first film and a second film that are sealed or bonded (*e.g.,* heat sealing, ultrasonic welding, chemical bonding, etc.) about at least a portion of their peripheries 1802. The first and second films define the interior reservoir of the body. Alternatively, the body 1810 may be formed from a single piece of material by injection molding, extrusion molding, blow molding, or other manufacturing processes known in the art.

In use, a female user applies pressure to the body 1806 which in turn raises the pressure within the body 1806, resulting in the fracture of the frangible seal 1804, and thereby expelling a dosage of the vaginal care composition out of the reservoir, through the plurality of dispensing openings of the insertion portion 1850 and onto the outer surface 1874 of the insertion portion 1850. The female user then may apply the vaginal care composition to the vaginal tissues of interest by swirling, pivoting, rotating, revolving, or gliding the insertion portion over/on the vaginal tissues of interest. In one example, the female user may squeeze and dose the vaginal care composition while simultaneously manipulating the applicator to apply the vaginal care composition to the vaginal introitus and optionally one or more external vaginal tissues.

### Elevator

Referring now to FIG. 3, an applicator 100, much like the applicator in FIG. 1, is depicted in cross-section. In this embodiment, the body 110 comprises a reservoir 102, a screw assembly 121, a screw base 123 (or external rotary grip), a spindle 114 having threads 116, and an elevator 118 that threadably engages the spindle 114. During use, a female user may advance the elevator 118 slideably toward the insertion portion 150 by rotating the screw base 123, which in turn rotates the spindle 114. Advancing the elevator 118 slideably toward insertion portion 150 will dispense a dosage of the vaginal care composition out of the reservoir 102, through the reservoir channel 108, and into the insertion portion, and out of the dispensing openings. Other mechanisms for advancing the elevator may be provided, such as a ratchet and pawl arrangement. Any means for reducing or preventing backflow may be disposed within the body, for example, between the reservoir 102 and the insertion portion 150, within the insertion portion 150, on the lateral outer surface of the insertion portion or any combination thereof, some examples of which are described below.

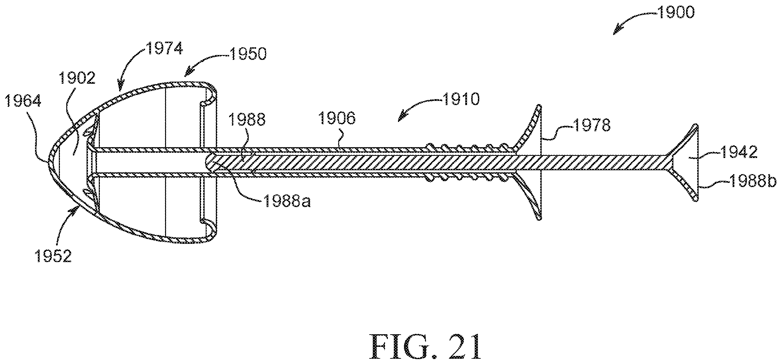

Referring now to FIG. 21, another non-limiting example of an applicator is depicted, where the applicator may be a single-use applicator. The applicator 1900 comprises an insertion portion 1950, wherein the insertion portion 1950 comprises a reservoir 1902 located within the insertion portion 1950, dispensing openings 1952, a tip 1964, and, optionally, a removable seal (not shown) that covers the dispensing openings. The applicator 1900 further comprises a rigid tube 1906 having a proximal end 1978, and an elevator stem 1988. The elevator stem 1988 has a base 1942. The elevator stem 1988 is slideably disposed at least in part within the rigid tube 1906, and extends longitudinally from the proximal end 1978 to the reservoir 1902. The elevator stem 1988 comprises a distal end 1988a that forms the elevator and a proximal end 1988b opposite the distal end 1988a, that forms the base 1942, which is graspable by the female user. In use, a female user removes the seal, if there is one, and slideably displaces the elevator stem 1988 towards the tip, which advances the distal end 1988a of the elevator towards the tip 1964 of the insertion portion 1950. Advancing the elevator stem 1988 toward insertion portion 1950 dispenses a dosage of the vaginal care composition from the reservoir 1902, out of the dispensing openings 1952 and onto the outer surface of the insertion portion 1950. In this embodiment, the insertion portion 1950 has a width that is greater than an outside width of the tube 1906. In some examples, the maximum width of the insertion portion is greater than the minimum outside width of the tube 1906. Reservoir 1902 may hold a sufficient quantity for a single dose without the need to refill, for example from about 0.1g to about 5g, or from about 0.25g to about 3g, or from about 0.5g to about 1.5g of vaginal care composition.

### Collapsible reservoir

Some applicators described herein may comprise a reservoir that can be collapsed when pressure is applied to the walls that form the reservoir. The user may collapse the reservoir by applying pressure directly to the walls forming the reservoir or by manipulating the applicator in a way that applies pressure to the walls that form the reservoir. In one example, the user may squeeze, pinch, or grasp the walls defining the reservoir with opposing pressure from two or more fingers to collapse the reservoir. In one example, the reservoir is collapsed due to a radial deformation of the reservoir walls of the body. The collapse of the reservoir causes the vaginal care composition stored within the reservoir to flow into structures in fluid communication with the reservoir.

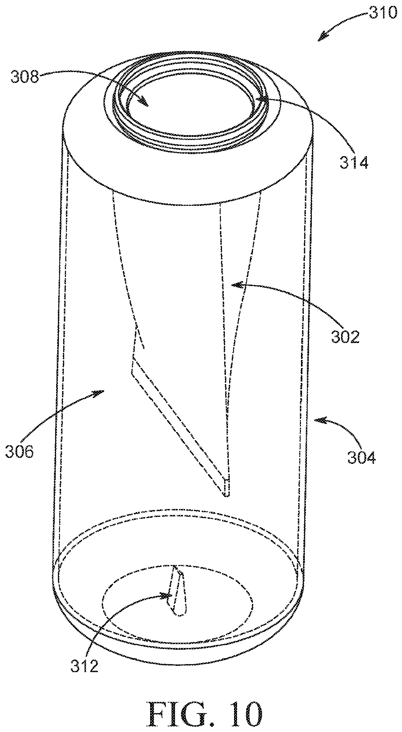

The collapsible reservoir may be contained in the body of an applicator and various bodies may be paired with various insertion portions described herein. Referring to FIG. 10, a body 310 comprises a male snap fitment 306 or other means for attaching an insertion portion to the body 310, such as threads. A shell 304 surrounds the reservoir 302, which stores the vaginal care composition. The reservoir 302 is collapsible and may be defined by or bounded by one or more flexible materials, such as a polymeric film, that forms one or more walls defining the reservoir. The shell may also formed from a flexible material. A cavity 307 filled with a gas is disposed between the reservoir 302 and the shell 304. The gas may be at atmospheric pressure. During use, a female user may apply hand pressure to the shell 304 which in turn raises the gas pressure above atmospheric pressure within the cavity 307 which in turn collapses the reservoir 302 to expel a dosage of the vaginal care composition from the reservoir though a channel 308 and into an insertion portion (not shown). A one-way valve 312 or vent (*e.g*., a slit valve) may be provided in the bottom of the shell 304 to allow the cavity to return to atmospheric pressure after a female user withdraws the hand pressure. This will also permit the shell to return to its pre-deformation state.

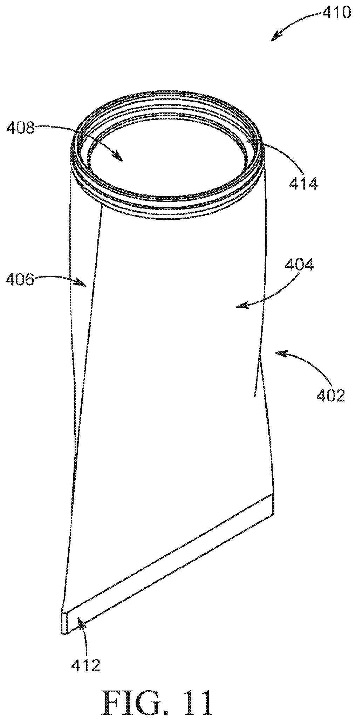

Referring now to FIG. 11 a non-limiting example of another body suitable for use with a variety of insertion portions is illustrated. The body 410 comprises a top or a neck having a snap fitment 406 thereat and a collapsible reservoir 402 storing a vaginal care composition therein. In some examples, the reservoir 402 may be defined or bounded at least in part by a first film 404 and a second film 409 that are sealed or bonded (*e.g.,* by heat sealing, ultrasonic welding, chemical bonding, etc.) about at least a portion of their peripheries 412. Alternatively, the reservoir 402 may be defined or bounded by a single piece of material by injection molding, extrusion molding, blow molding, or other manufacturing processes known in the art. The walls defining the reservoir 402 may be formed using any suitable material including plastics and metals like aluminum. During use, a female user may apply hand pressure to expel a dosage of the vaginal care composition through reservoir channel 408 and into an insertion portion (not shown), and out of the dispensing openings of the insertion portion.

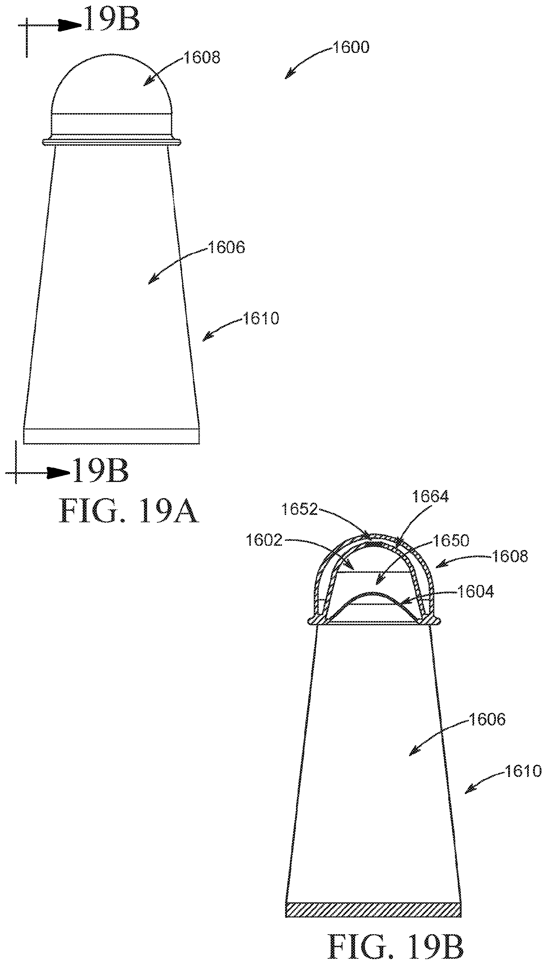

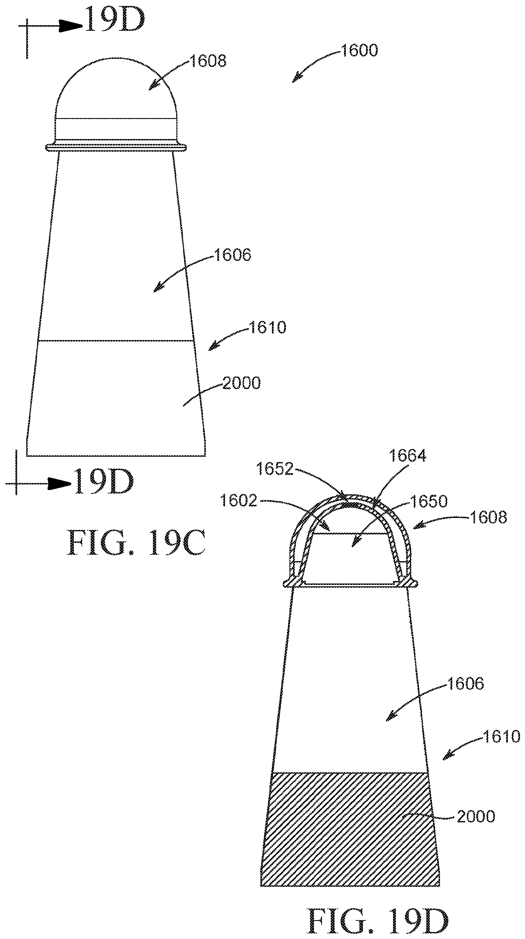

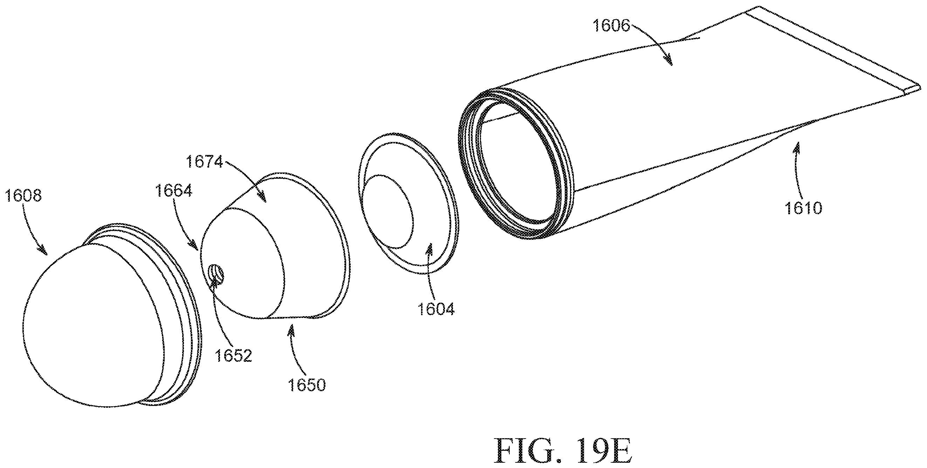

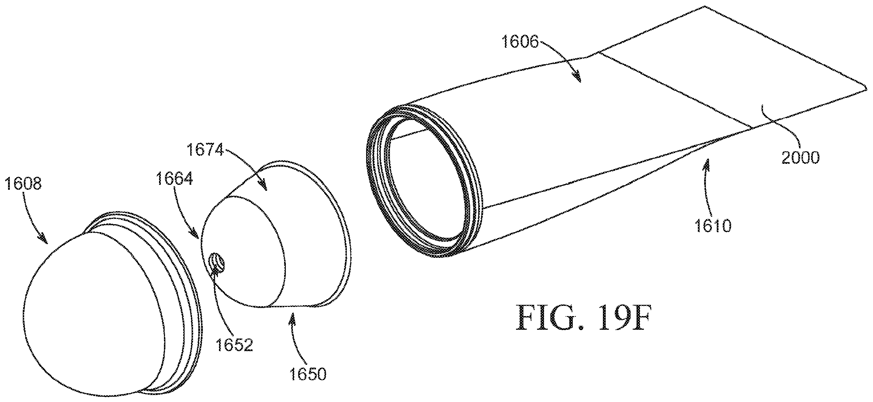

Examples of collapsible reservoirs are shown in FIGs. 5, 10, 11, and 19A-19F.

### Means for Reducing Backflow

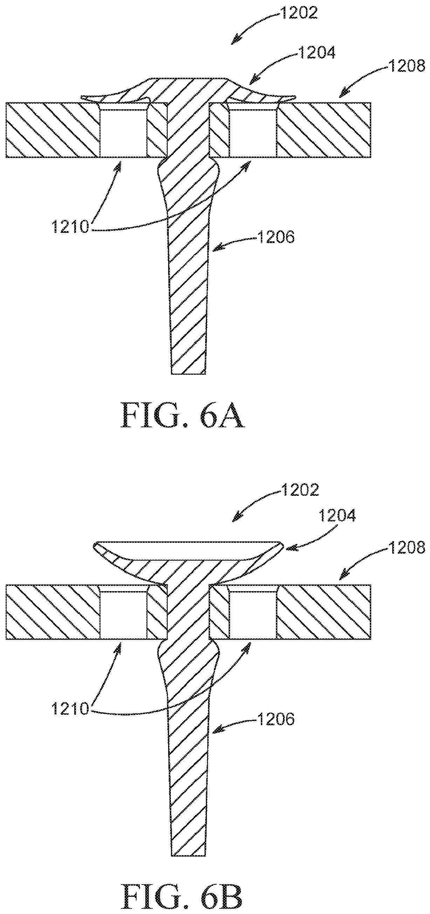

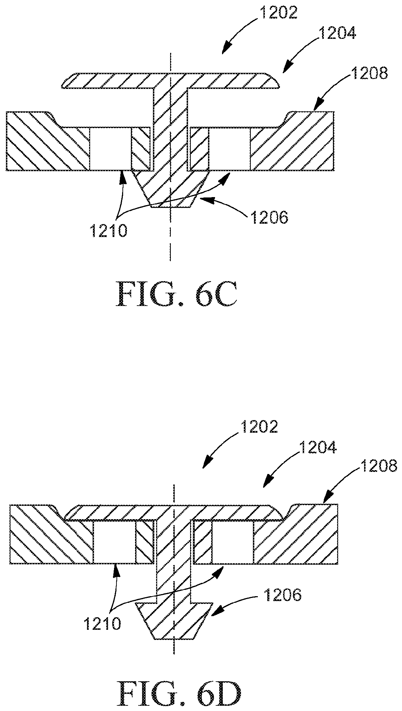

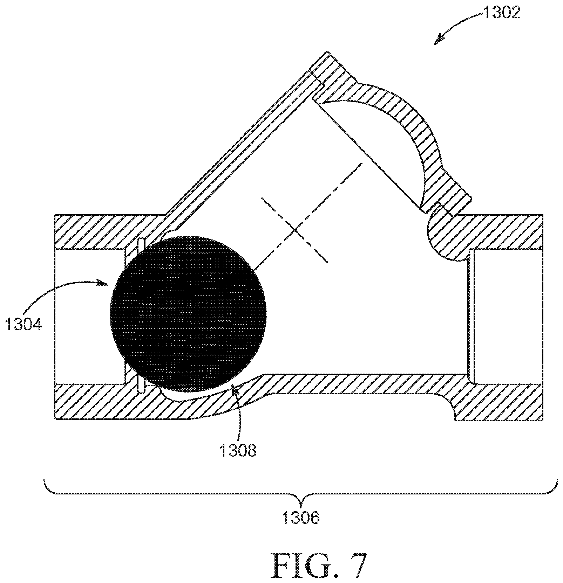

FIGS. 6 and 7 illustrate examples of one-way valves that may reduce or prevent backflow of fluids into passages upstream of these valves and/or the reservoir of an applicator. These valves may be used with one or more of the applicators described herein. These valves may be used in combination with a separate pump or other means to dispense the vaginal care composition from a reservoir. While the specific implementation of each valve is not shown within the full context of an applicator, one of ordinary skill in the art will appreciate how the alternative valves can be used to regulate the flow of a vaginal care composition between the reservoir and the dispensing openings.

Referring now to FIGS. 6A and 6B, a non-limiting example of a flexible umbrella valve 1202 is shown in a closed position and in an open position, respectively. Flexible umbrella valves 1202 may be made of elastomeric materials (e.g., rubber). Referring now to FIGS. 6C and 6D, a non-limiting example of a rigid umbrella valve 1202 is shown in an open position and in a closed position, respectively. The rigid umbrella valves 1202 may be made of rigid materials (e.g., polyethylene or polypropylene). In both flexible and rigid umbrella valves, the valve components comprise an umbrella or mushroom shaped disk 1204 for providing sealing and a stem 1206 for proper seating. Umbrella valves, in general, are used for sealing purposes in devices where it's desirable for backflow of a fluid to be reduced or prevented. In FIG. 6A and 6B, the disk 1204 is shaped like a convex diaphragm, which is configured to be positioned flush with the surface 1208 or on a seat that is recessed from the surface 1208. The convex portion of the disk 1204 conforms to the surface of the seat 1208, as shown in FIG. 6A.

For a flexible umbrella valves, a pressure head of the vaginal care composition above a predefined threshold flexes the valve raising the convex portion of the disk 1204 to allow the vaginal care composition to flow through the holes 1210 positioned on the seat 1208 in a forward direction, as shown in FIG. 6B. However, the disk 1204 reduces or prevents backflow of the vaginal care composition when a reverse backflow pressure is experienced on an upper surface of the disk 1204.

For a rigid umbrella valve, a pressure head of the vaginal care composition above a predefined threshold translates the valve raising the disk 1204 to allow the vaginal care composition to flow through the holes 1210 positioned on the seat 1208 in a forward direction, as shown in FIG. 6C. However, the disk 1204 reduces or prevents backflow of the vaginal care composition when a reverse backflow pressure is experienced on an upper surface of the disk 1204. The holes 1210 of the valve may be flush with the seat 1208 (e.g., FIG. 6B) or recessed below the seat 1208 (e.g., FIG. 6C).

Referring now to FIG. 7, a non-limiting example of a ball valve 1302 is shown. Typically, a ball valve opens and closes by rotation or movement of a ball, where the ball has a port such that when the port is in line with ends of the valve, to allow the flow of a fluid. Another example of the ball valve describes a ball which has no ports, but gets displaced from a seat based on pressure of the incoming fluid and returns to the seat under normal operating pressure, thereby reducing or preventing backflow of the fluid. In general, ball valve 1302 reduces or prevents the flow of a liquid in one direction by making use of a sphere 1304, or commonly referred as a ball, positioned within the ball valve 1302. The sphere 1304 is seated in a valve seat 1308, where the sphere 1304 is displaced when the pressure of the vaginal care composition exceeds a pressure level and returns to the seating when the pressure is below a lower threshold.

A means for reducing or preventing backflow may be provided as an isolation or shut-off valve (not shown), which have open and closed positions. Isolation valves typically employ a disc, washer, or gasket that engages a seat to prevent fluid flow in both directions when closed. Other examples of isolation valves include gate valves, which incorporate a sliding gate to block fluid flow, and globe valves in which a plug is moved directly on and off the valve seat in a manner to selectively allow fluid flow.

Other means for reducing or preventing backflow include 1-way valves, such as a duckbill valve, a butterfly valve, a diaphragm valve, a slit valve, and one-way swing valve may also be used with the applicators herein. A duckbill valve (not shown) is a valve that is duck-beak shaped which prevents backflow of a fluid. A butterfly valve is a flow control valve which includes a rotational disk that controls a fluid. It usually consists of a body, liner, shaft and disk assembly for insertion within a conduit, where the valve seals off the flow completely at one end of its rotation and allows maximum flow through the conduit at the other end of its rotation. A diaphragm valve (not shown) is a valve that utilizes a resilient diaphragm that engages a weir to control flow of fluid past the weir, wherein the diaphragm may be controllably lifted and sealed against the weir to selectively permit flow through the valve. A slit valve (not shown) is a one-way valve formed by one or more slits cut into a flexible barrier. When the fluid pressure on the fluid side of the barrier is greater than the fluid pressure outside the barrier by a predefined amount, an opening defined by the slit expands to allow the fluid flow, and reduces or prevents fluid flow when the pressure is below the predefined amount.

A 1-way valve may be provided in the form of a metering valve between the reservoir and the dispensing openings. A metering valve regulates the amount or dosage of the vaginal care composition that is dispensed through the dispensing opening(s). A metering valve typically comprises a dosage chamber fillable by a fixed amount of the vaginal care composition, which is then pushed out of the dosage chamber by actuation of plunger or other means for generating pressure to expel the dosage of vaginal care composition in the dosage chamber, as known in the art. The metering valve may have a dosage chamber capable of holding from about 0.1 to about 2g, or from about 0.1 to about 1.5 g, or from about 0.5 to about 1.2 g of vaginal care composition. The metering valve may enable a user to easily dose an amount of vaginal care composition. The metering valve may be adjusted by the user to modify or customize the amount of vaginal care composition dosed per actuation of the metering valve.

More than one valve and/or a combination of different valves may be provided *(e.g.,* a metering valve and another type of 1-way valve). Or, one valve may perform multiple functions.

In some instances, a valve, such as a one-way valve, may be located at the dispensing opening (*e.g.,* 152 in FIG. 1) at or near the lateral, outer surface *(e.g.,* 174 in FIG. 1) of an insertion portion. For example, the valve might form a part of the dispensing opening itself, wherein the valve opening (*e.g.,* a slit in the case of a slit valve) functions as the dispensing opening in the outer surface or, in other instances, the valve might be located just upstream (*e.g*., 0-2 mm) of the dispensing opening. In some instances, each dispensing opening may have a corresponding valve to reduce or prevent backflow of a fluid upstream of the dispensing opening. In other instances, each of the dispensing openings may have a one-way valve associated therewith at or near the lateral, outer surface, and the applicator may further incorporate a metering valve upstream of the dispensing openings and/or downstream of the reservoir. The one-way valve may be smooth and free of sharp edges and crevices. In some instances, the one-way valve located at or near the lateral, outer surface may be a slit valve or an umbrella valve. The insertion portion may have a single dispensing opening with a single one-way valve or multiple dispensing openings with multiple one-way valves. Locating the one-way valve on or near to the lateral, outer surface may further minimize the amount of contamination downstream of the dispensing openings.

Referring now to FIGS. 4 and 5, an applicator comprising a body 210, which comprises an aerosol valve 1002 and a reservoir 202 comprising a vaginal care composition, is illustrated. The body 210 comprises an aerosol valve 1002 in communication with the reservoir 202 having a vaginal care composition disposed therein. The aerosol valve 1002 may also be a metering valve and may also function as a 1-way valve, although aerosol valves which are not metering and do not function as a 1-way valve are also suitable for use with the applicators disclosed herein. The aerosol valve 1002 is disposed between an insertion portion, which may be removably, integrally or fixedly attached to/connected to/formed with the body, and the reservoir 202. The body 210 further comprises a rigid outer shell 220 and a cavity 204 disposed between the shell 220 and one or more walls that define the reservoir 202. The shell may substantially surround the reservoir. The cavity 204 may comprise a pressurized gas (which may be pressurized during manufacture or a pump may be provided that is actuated by the user) and the reservoir may be collapsible. In other embodiments, a propellant is mixed with vaginal care composition in the reservoir 202, which may be provided as either collapsible or rigid. The reservoir can be collapsed when the aerosol valve is actuated and the pressure within the cavity that is applied to one or more walls that define the reservoir is greater than the pressure within the reservoir. The collapse of the reservoir causes the vaginal care composition stored within the reservoir to flow into structures in fluid communication with the reservoir. The vaginal care composition may comprise a propellant. During use, a female user may expel a dosage of the vaginal care composition from the reservoir 202 through the channel 1020 of the aerosol valve 1002 and into an insertion portion by depressing the insertion portion, which in turn depresses slideable stem 1004.

The aerosol valve 1002 may be attached to the upper end of the reservoir 202 or elsewhere on the body between the reservoir 202 and an insertion portion. In some examples, the insertion portion 150 engages the stem 1004 via a snap or friction fit. The aerosol valve 1002 generally comprises a spring actuated slideable stem 1004, which moves in a vertical direction. The slideable stem 1004 is biased by a spring 1010. The aerosol valve 1002 further comprises a mounting cup 1006 and a housing 1008 positioned inside the mounting cup 1006. The slideable stem 1004 is slideably disposed within an upper opening 1006a of the mounting cup 1006 and extends axially through the housing 1008. The spring 1010 is configured to seat against the slideable stem 1004. A dosage chamber 1012 is positioned below an upper inner surface of the mounting cup 1006 and within the housing 1008.

The aerosol valve 1002 further comprises one or more seals, such as a gasket 1014 and a lip gasket 1016, positioned on opposing ends of the dosage chamber 1012. The internal gasket 1014 and the lip gasket 1016 are configured to seal the dosage chamber 1012. The aerosol valve 1002 also comprises a gasket 1018, which provides sealing between the housing 1008 and the body of the body. The slideable stem 1004 of the aerosol valve comprises a channel 1020 which axially extends along at least a portion of the length of the slideable stem 1004. A hole 1022 is positioned on the slideable stem 1004, which provides for fluid communication between the dosage chamber and the channel 1020 when the stem is displaced downward. Further, the slideable stem 1004 also comprises a wedge portion 1024 that allows the lip gasket 1016 to open and close based on the axial movement of the slideable stem 1004.

In use, displacement of the slideable stem 1004 by an insertion portion opens the lip gasket 1016 and allows passage of the pressurized vaginal care composition from the reservoir 202 to the aerosol valve, namely the dosage chamber 1012 of the aerosol valve, which may be in fluid communication with a conduit 162 (see FIGS. 2A and 2B), one or more channels 158, 160, and the dispensing openings 154, 156 of an insertion portion 150.

The axial plunging of the slideable stem 1004 also causes the hole 1022 positioned on the body of the slideable stem 1004 to be displaced beyond the internal gasket 1014. The displacement of the hole 1022 beyond the internal gasket 1014 permits the pressurized vaginal care composition to exit the dosage chamber 1012 via the channel 1020 of the slideable stem 1004. The channel 1020 is in fluid communication with the channel(s) of the insertion portion and the dispensing openings. Other configurations of aerosol valves may be provided.

### Additional Applicators

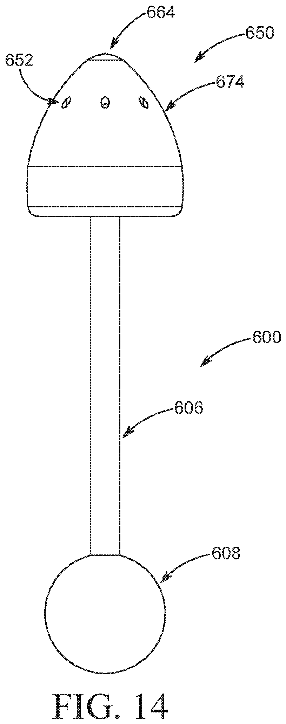

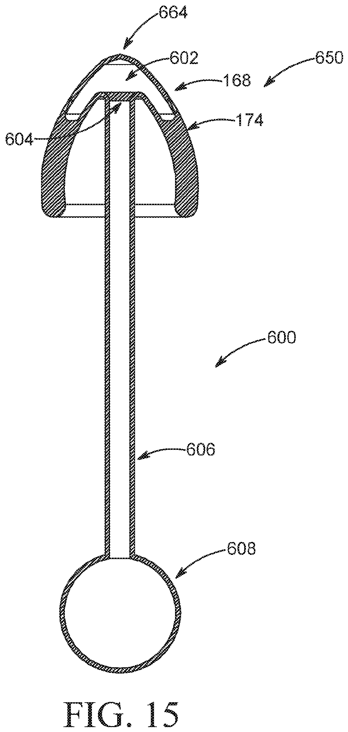

Referring now to FIGS. 14 and 15, another non-limiting example of an applicator is illustrated. Applicator 600 comprises a collapsible chamber 608 having a gas (*e.g*., air) disposed therein, a tube 606 and frangible seal 604 (as shown in FIG. 15). The gas fills the collapsible chamber 608 and the tube up to the frangible seal 604. The applicator 600 further comprises an insertion portion 650 comprising a reservoir 602 having a vaginal care composition disposed therein. The insertion portion 650 may have as a size and/or outer shape the same as, or similar to, any of the insertion portions described herein. While a single, circumferential row of dispensing openings 652 are shown in FIG. 14, other arrangements of the dispensing openings may be provided. The dispensing openings 652 are in fluid communication with the reservoir 602.

The frangible seal 604 may be provided in a form known in the art, such as, for example, a perforated, serrated, or pre-stressed film which will fracture and open upon application of pressure. While the frangible seal is shown disposed at the distal end of the tube 606 (*i.e*., at the exit of the tube 606) and adjacent the reservoir of the insertion portion 650, the frangible seal may be located elsewhere. For example, the frangible seal 604 may be located at the exit of the collapsible chamber 608 or the entrance of the tube 606.

A removable seal (not shown) may be provided that covers the dispensing openings 652, as previously described in connection with other embodiments. The seal may be formed from a flexible film or provided as a rigid or flexible cap.

In use, a female user applies pressure to the collapsible chamber 608 which in turn raises the pressure within the collapsible chamber and the tube, resulting in the fracture of the frangible seal 604. The gas escaping from the frangible seal then pushes a dosage of the vaginal care composition out of the reservoir 602, through the dispensing openings 652 and onto the outer surface 674 of the insertion portion 650. The female user then may apply the vaginal care composition to the vaginal tissues of interest by swirling, pivoting, rotating, revolving or gliding the insertion portion over/on the vaginal tissues of interest.

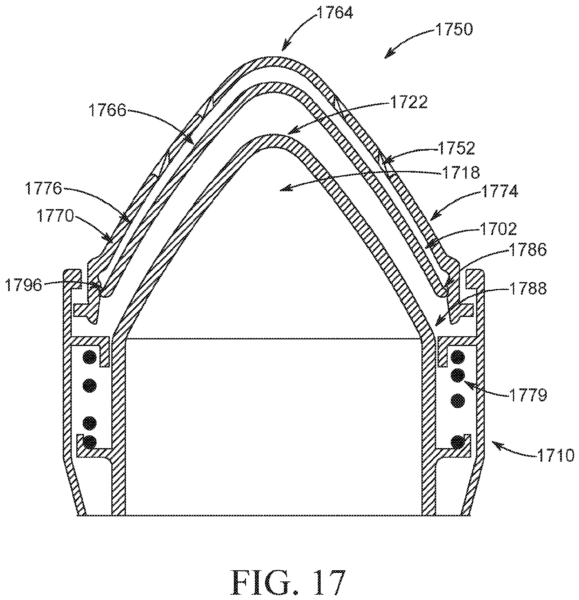

Referring now to FIG. 16, another applicator is illustrated. The applicator 1700 comprises an insertion portion 1750, a removable seal 1768, and a body 1710. The body 1710 comprises a sleeve 1720 having a button 1704 positioned at the base of the sleeve 1720 opposite the insertion portion 1750. When a user depresses the button 1704, it in turn displaces a piston 1718 (which may comprise a cone shaped tip 1722) slideably disposed within the sleeve 1720 toward the insertion portion, which in turn displaces a slideably disposed plunger 1766 (*see, e.g.,* FIG.8 and 17) of the insertion portion 1750 toward the tip 1764 to expel a dosage of the vaginal care composition stored within the reservoir 1702 (shown subsequently in FIG. 17) of the insertion portion 1750 through the plurality of dispensing openings 1752.

The removable seal 1768 may cover the dispensing openings 1752. In some examples, the seal 1768 may cover the entire outer surface of the insertion portion. The seal may be formed from a flexible film or provided as a rigid or flexible cap with or without the perforations 1770.

Referring now to FIG. 17, the insertion portion 1750 and a portion of the body 1710 of FIG. 16 are shown in engagement. The reservoir 1702 is defined by the upper surface of the plunger 1766 and the inner surface 1776 of the wall 1770. In some examples, the plunger 1766 may be rigid and provided in a cone shape. In some embodiments, the shape of the tip 1722 of the piston 1718 may be the same as or similar to the shape of the plunger 1766 (*e.g*., cone-shaped). The plunger 1766 may further comprise a lip 1786 located on the outer surface of the plunger 1766 that engages a recess of the inner surface 1796 of the wall 1770 after the plunger 1766 is moved longitudinally toward the tip 1764 by the piston tip 1722. In another example, the plunger 1766 may be fixedly attached to or integrally formed with the wall 1770 at the proximal end 1778 of the insertion portion 1750. At least a portion of the plunger 1766 may be formed from a flexible or stretchable material, such as an elastomeric film, that may be displaced toward the insertion portion tip 1764 by displacement of the piston 1718. This movement in turn decreases the volume of the reservoir 1702 thereby expelling a dosage of the vaginal care composition through the dispensing openings 1752 and onto the outer surface 1774 of the wall 1770. In another example, the insertion portion 1750 may be used as a standalone applicator apart from the body 1710, wherein a female user may dispense the vaginal care composition from the reservoir 1702 and through the dispensing openings 1752 by applying finger pressure to the plunger 1768 to displace the plunger 1768 toward the tip 1764. The female user may due this while grasping the applicator at its proximal end 1778.

The wall 1770 of the insertion portion 1750 may be shaped and sized to accommodate the anatomical geometry of the vaginal introitus and for applying the vaginal care composition thereto. The wall 1770 may have a size and/or outer shape the same as or similar to any of the insertion portions described herein. While a single, circumferential row of dispensing openings 1752 are to distribute the dosage of the vaginal care composition about the outer surface, other arrangements may be provided. The reservoir may be sized to hold from about 0.1g to about 5g, from about 0.25g to about 3g or from about 0.5g to about 1.5g of a vaginal care composition.

The various applicators described herein may be configured to regulate an amount of the vaginal care composition dispensed therefrom, such that the amount of the vaginal care composition released by the body may be a single dose consistent with the above-described ranges. For example, the vaginal care composition can be released by a turn or a click by metered dosing or squeezing or grasping or manipulating the applicator. In other embodiments, the amount dispensed per actuation of a pump may be such that single actuation or multiple actuations are needed to dispense an amount consistent with the above-described ranges.

The vaginal care composition can be dispensed via the user actuating a pump (e.g., see FIG 12), continuously rotating a knob or portion of the applicator (e.g., see FIG 3), a discrete rotation of a portion of the applicator, e.g., a knob, pushing or sliding a button, or dispensing through a metering valve. In one example, a continuous rotation is used and a 360-degree rotation dispenses from about 0.5g to about 10g, or from about 0.75g to about 7.5g, or from about 1g to about 5g, or from about 2g to about 4g of vaginal care composition. A discrete rotation or actuation may be used to dispense from about 0.1g to about 5g, or from about 0.2g to about 2g, or from about 0.25g to about 1g, or from about 0.3g to about 0.5g of the vaginal care composition. When the discrete actuation is rotational, each actuation may be set to be about 30°, about 45°, about 60°, about 90°, about 120°, about 180° or about 360°. Each actuation may dispense a fraction of a dose, such as ¼, ⅓ or ½ of a dose, or a whole dose of the vaginal care composition.

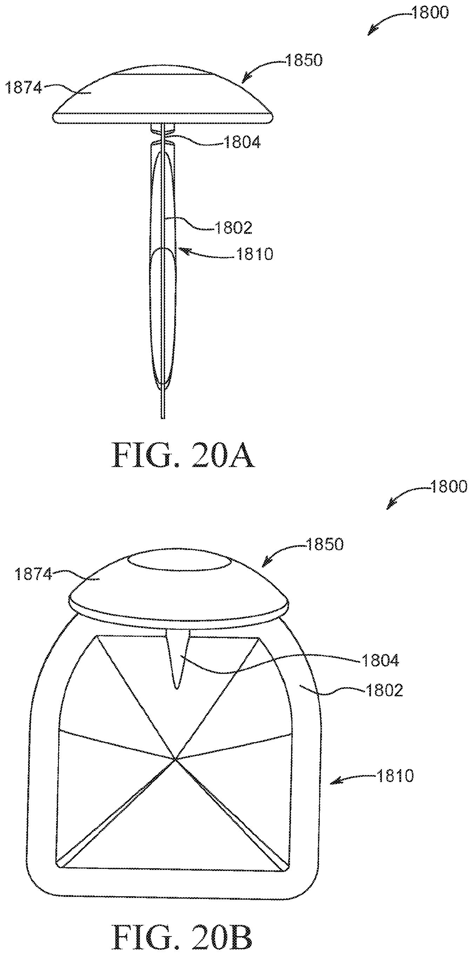

Some non-limiting shapes for the applicators have been described and shown herein. Other shapes and/ or sizes may also be provided. For example, an applicator comprising an insertion portion and body may have an outer shape and/or dimensions as described in one or more of U.S. Provisional Application Serial Nos. 62/622,280, entitled "Applicators For Treating Vaginal Dryness" filed on January 26, 2018; 62/622,289, entitled "Kits For Treating Vaginal Dryness" filed on January 26, 2018; and 62/622,298, entitled "Methods For Treating Vaginal Dryness" filed on January 26, 2018. In some instances, the applicators described herein may have a shape and/or size as shown in one or more of FIGS. 2, 3, 7 and/or FIGS. 9A to 21 of U.S. Provisional Application Serial No. 62/622,298 and the corresponding written description therein (*e.g*., pages 19-28 thereof).

In some examples, one or more portions of the applicator may be formed in whole or in part from a thermoplastic elastomer, a natural rubber, metal (e.g., aluminum, stainless steel), a synthetic rubber (*e.g.,* a silicone elastomer/silicone rubber), a polyester (*e.g.,* a polyurethane, such as STERALLOY^{™} 2021-5 available from Hapco, Inc.) and/or a thermoplastic. In some examples, the material may have a hardness form about 5 Shore A to about 80 Shore D, or from about 10 Shore A to about 100 Shore A, or from about 15 Shore A to about 70 Shore A. The material used to form the applicator is preferably medical grade. Some examples of suitable thermoplastics include polypropylene, high density polyethylene, low density polyethylene, polystyrene, polyethylene terephthalate, acrylonitrile butadiene styrene, nylon, polycarbonate, and polyacrylic. The dispensing openings (*e.g*., 152) will provide a smooth surface, and are shaped, sized and oriented to allow for comfortable use. In some examples, the insertion portion is an open cell foam.

While various arrangements of insertions portions and bodies have been described and shown herein, other arrangement may be provided. For example, features or structures described as part of an insertion portion may be disposed instead on a body or vice versa. For example, a frangible seal or diaphragm described as part of a body may be located on an insertion portion instead. Further, a structure or feature described as part of an insertion portion or a body may be provided as an intermediary structure or feature disposed between an insertion portion and a body. Still further, a body or insertion portion may contain more or less than all of the features or structures shown or described as part of a non-limiting example of the present disclosure.

While specific examples of various insertion portions, backflow prevention means, and bodies have been described above, it is understood that alternatives that operate in a similar or different manner may be contemplated by one skilled in the art without departing from the scope of the application.

### B. Vaginal Care Compositions

The reservoir may store a wide variety of vaginal care compositions suitable for application to the vaginal introitus and, optionally, external vaginal tissues for treating vaginal dryness, vaginal irritation, vaginal itch, vaginal infection, vaginal odor, vaginal chafing, dyspareunia, and/or vaginal atrophy. In some examples, the vaginal care compositions are preferably non-irritating and substantially free of ingredients that are less suitable for application to these tissues. The vaginal care composition may be provided, for example, in the form of a spreadable gel, serum, lotion, paste, foam, or cream. Some non-limiting examples of vaginal care compositions include vaginal moisturizing compositions (*e.g*., REPLENS^{®}, NEOGYN^{®}, VAGISIL^{®} PROHYDRATE^{®}, and ESTRACE^{®}), antifungal compositions (*e.g.,* MONISTAT^{®} Vaginal Antifungal), antibiotic compositions (*e.g*., CLEOCIN^{®} Vaginal Cream), itch relief compositions (*e.g*., MONISTAT^{®} Instant Itch Relief Cream and VAGISIL^{®} Maximum Strength Anti-Itch Creme), anti-chafing compositions (*e.g*., MONISTAT^{®} Chafing Relief Powder Gel and BODY GLIDE^{®} SKIN GLIDE^{®} Anti Chafing Cream), anti-irritation compositions (*e.g*., BALMEX^{®} Adult Care Rash Cream and DESITIN^{®} Rapid Relief Cream), odor control formulations (*e.g*., REPHRESH^{®} Vaginal Gel and MONISTAT^{®} Stay Fresh Gel), and vaginal lubricant compositions (*e.g*., K-Y^{®} UltraGel and Jelly, ASTROGLIDE^{®}, WET PLATINUM^{®}, ALLATION^{®} Gentle Moisture, and PURE ROMANCE^{®} Just Like Me). In some examples, the vaginal care composition provides a moisturization and/or hydration benefit to one or more vaginal tissues. In some examples, the vaginal care composition may comprise an estrogen agent and/or a progesterone agent.

While the applicators and methods described herein are suitable for use with any vaginal care composition, the applicators and methods described herein may be particularly useful for treating or ameliorating vaginal dryness, vaginal irritation, vaginal itch, vaginal infection, vaginal odor, vaginal chafing, vaginal atrophy, and/or other conditions, for which treatment over an extended period of time (*e.g.,* daily use over multiple days or weeks) is desirable. In some examples, applicators that are hygienic would be preferable to ensure consumer habit adoption and long-term use to improve such conditions. Hygienic applicators may be especially preferred by consumers who may be more susceptible to infections in the vaginal area (*e.g.,* urinary tract infections). Applicators with one or means for preventing backflow of fluids may enable a hygienic consumer experience by reducing or preventing contamination due to backflow of fluid into upstream fluid passages and/or the reservoir. This includes reducing or preventing the backflow of cleaning solutions (*e.g*., soap and water), bodily fluids, and vaginal care composition that has come in contact with the user's introitus and/or external vaginal tissues.

Without wishing to be bound by theory, it is believed that vaginal dryness and/or atrophy may be best treated by applying a vaginal care composition suitable for treating or ameliorating vaginal dryness and/or atrophy to the user's vaginal introitus and, optionally, one or more external vaginal tissues over an extended period of time (*e.g*., daily use over multiple days or weeks). Applicators with reservoirs capable of storing one or more doses of vaginal care composition may increase consumer compliance over extended periods of time by making the process more convenient and intuitive.

A non-limiting description of vaginal care compositions which may be suitable for use in treating or ameliorating vaginal dryness and/or vaginal atrophy will now be described. These vaginal care compositions may be provided in the form of oil-in-water emulsions with one or more additional ingredients may be preferred in some instances. An oil-in-water emulsion may provide a sensorial feel that is light and non-greasy, but still delivers moisturization and lubricity without aesthetic negatives like stickiness or heavy residue. Further, the vaginal care compositions may further comprise one or more vaginal care agents. The vaginal care composition may comprise one or more water soluble ingredients.

In some examples, vaginal care compositions for treating vaginal dryness and/or vaginal atrophy may comprise water, one or more oils, and, optionally, one or more vitamins and/or pro-vitamins (*e.g*., a substance that may be converted to a vitamin by metabolic processes), one or more thickeners, one or more emulsifiers, one or more humectants, one or more lubricants (which may also be in the form of an oil), one or more moisturizers (which may also be in the form of an oil), one or more feel modifiers (*e.g*., particulates, powders, and film forming agents), one or more preservatives, and one or more pH adjusting agents. The vaginal care composition preferably provides a suitable viscosity, dry feel, moisturization/emolliency benefits, suitable lubricity and/or vaginal skin health benefits.

The vaginal care composition may comprise water in an amount greater than 50%, or greater than 60%, or from about 60% to about 90%, or from about 60% to about 80%, or from about 65% to about 75% by weight of the vaginal care composition. The water may provide a carrier for water soluble ingredients as well as a moisturization benefit. The vaginal care composition may comprise one or more oils, including botanical oils, silicone oils and other oils. The vaginal care composition may comprise less than about 30%, or from about 1% to about 20%, or from about 5% to about 15%, or from about 10% to about 15% by weight of the vaginal care composition of oil(s). In some examples, the botanical oil is derived from one or more plant source materials, such as leaf, root, bark, stem, flower or seed of a plant. The botanical oil may be a seed oil, a nut oil, a flower oil, or a leaf oil. The botanical oil may comprise polyunsaturated fatty acids, preferably omega-3 (*e.g*., α-linolenic acid) and/or omega-6 fatty acids. The botanical oil may be a coconut oil, a sunflower seed oil, a safflower oil, and combinations thereof, which may be rich in omega-3 and/or omega-6 fatty acids. The botanical oil may provide an emolliency vaginal skin care benefit. The vaginal care composition may comprise from about 0.1% to about 2%, or from about 0.2% to about 1%, or from about 0.2% to 0.5% by weight of the vaginal care composition of botanical oil(s).

The vaginal care composition may comprise one or more vitamins and/or pro-vitamins for providing a vaginal skin health benefit. Some non-limiting examples include niacinamide, panthenol, vitamin B3, vitamin B5, vitamin E, and derivatives thereof. The vaginal care composition may comprise from about 0.1% to about 7%, or from about 0.5% to about 5%, or from about 2% to about 4% by weight of the vaginal care composition of vitamin(s) and/or provitamin(s).

The vaginal care composition may further comprise one or more silicone oils. Silicone oils are liquids at ambient conditions comprising one or more polymerized siloxanes or silicone polymers (*e.g*., polysiloxanes, polydimethylsiloxanes (PDMS), and combinations thereof). The silicone oil may comprise dimethicone, dimethiconol (a high molecular weight silicone gum), and combinations thereof, one example is Dow Corning^{®} 1503 Fluid which comprises a combination of dimethicone and dimethiconol. The vaginal care composition may comprise from about 0.1% to about 4%, or from about 0.5% to about 3%, or from about 1% to 3% by weight of the vaginal care composition of silicone oil(s).

The vaginal care composition may comprise one or more humectants, such as glycerol. The humectants may also provide a moisturizing benefit. The humectant may be provided in an amount from about 5% to about 20%, or from about 5% to about 15%, or from about 8% to about 12% by weight of the vaginal care composition. Other humectants which may be provided include other polyhydroxy alcohols (*e.g*., sorbitol, propylene glycols, butylene glycols, pentylene hexylene glycols), polyethylene glycols, aloe vera, in any of its forms, hyaluronic acid, and combinations thereof. The vaginal care composition may further comprise one or more solid micro-particles, such as silicone particulates/silicone powders, to impart desirable feel characteristics to the vaginal care composition. In some embodiments, the vaginal care composition comprises siloxane particles (*e.g*., polymethylsilsesquioxane) having an average particle size from about 1µm to about 15µm. The particles may be in the form of mono-disperse microspheres. The polymethylsilsesquixane particles are sometimes also referred to as silicone resins. Some non-limiting examples of polymethylsilsesquioxane particles include TOSPEARL^{®} series from Momentive Performance Materials, Inc., including TOSPEARL^{®} 2000, TOSPEARL^{®} 145, TOSPEARL^{®} 150, TOSPEARL^{®} 1320 and the like. The small particle size and nature of the particles may impart a dry feel initially or when touched by the user (which provides a beneficial initial experience). The ball bearing effect may also facilitate the ease of spreading, rubbing, or otherwise applying the vaginal care composition to the vaginal tissues of interest.

The vaginal care composition may further comprise one or more emulsifiers, and/or one or more thickeners. Some non-limiting examples of emulsifiers include cationic surfactants, anionic surfactants, non-ionic surfactants, polyethylene glycol, polyethylene glycol polypropylene copolymers, alkyl glucosides, and fatty alcohols. Some non-limiting examples of thickeners include gums, starches, modified starches, clays, cross-linked water swellable polymers, cetearyl glucoside, cetearyl alcohol, behenyl alcohol, cetyl alcohol, stearyl alcohol, polyacrylates, and polyacrylamide. The thickeners are provided in amounts to facilitate achieving the desired viscosity in combination with the other ingredients. The thickeners may be provided in an amount from about 1% to about 10%, or from about 2% to about 8%, or from about 4% to about 8% by weight of the vaginal care composition.

The vaginal care composition may preferably have a viscosity suitable for dispensing onto the outer surface of the insertion portion without dripping or runniness as the applicator is manipulated by a user prior to spreading the vaginal care composition about the vaginal introitus and/or external vaginal tissues. The vaginal care composition may also have a viscosity conducive to spreading onto the vaginal tissues of interest using the applicators described herein without undue effort. In some examples, it may be desirable for the vaginal care composition to be dispensable from a pump type dispenser. In some examples, the vaginal care composition may exhibit a viscosity of from about 2,000 cps to about 200,000 cps; in some examples, from about 5,000 cps to about 150,000 cps; and in some examples, from about 20,000 cps to about 90,000 cps or any integer value from about 2,000 cps to about 200,000 cps, or any range formed by any of the preceding values.

In some examples, the vaginal care composition may be substantially free of retinol, retinyl esters, retinaldehyde, peptides, ethanol, sunscreens, and sensates. In some examples, the composition may be substantially free of perfumes and pigments. In some examples, the composition may be substantially free of particulates for exfoliation. In such examples, the excluded particulates have an average particle size of from about 125 microns to about 700 microns or more. Examples of such particulates may include polyethylene terephthalate (PET) microbcads, crushed apricot kernel shells, salt crystals, sugar crystals, and crushed volcanic rock. It is believed the foregoing ingredients may either be irritating or present an unsatisfactory user experience in a vaginal care composition. In some examples, the vaginal care composition may be substantially free of the combination of carbomer and polycarbophil and other bioadhesives or mucoadhesive ingredients.

### C. Methods of Use (not claimed)

The applicators may be used in a variety of ways. In one example, a method for treatment of one or more of vaginal dryness, vaginal irritation, vaginal itch, vaginal infection, vaginal odor, vaginal chafing, dyspareunia, and/or vaginal atrophy using an applicator - a multi-use applicator or a single-use applicator - is provided. A female user suffering from one or more of the vaginal conditions grasps an applicator - a multi-use applicator or a single-use applicator - that comprises a reservoir, a vaginal care composition stored within the reservoir, and, optionally, one or more dispensing openings in an outer surface of the applicator that are in fluid communication with the reservoir. In some examples, the reservoir is configured to hold the vaginal care composition for a plurality of days without refilling the reservoir. In other examples, the reservoir holds a sufficient amount for a single use only. The female user dispenses a dosage of the vaginal care composition from the reservoir, onto an outer surface of the applicator. The dosage may be dispensed by collapsing, at least in part, the reservoir. The applicator may comprise a reservoir with a vaginal care composition therein and an elevator slideably disposed within the applicator. The female user may dispense the dosage by advancing the elevator slideably disposed within the applicator. The female user may administer the dosage or a portion thereof to her vaginal introitus and, optionally, one or more external vaginal tissues, while grasping the applicator.

In examples where the applicator comprises a removable insertion portion, a female user may grasp the applicator comprising a body having an attachment structure. The female user may removably attach an insertion portion to the body by engaging the attachment structure. The female user may dispense a dosage of a vaginal care composition onto an outer surface of the insertion portion, and the female user may administer at least a portion of the dosage to her vaginal introitus and, optionally, one or more external vaginal tissues, with the insertion portion of the applicator.

In some examples, the method further comprises the female user disposing of the single-use applicator after administering the portion of the dosage. For example, the applicator may be thrown in the trash following its use. In some examples, the method further comprises the female user repeating steps of grasping, dispensing, and administering using a new single-use applicator. In some examples, the method further comprises the female user selecting each new single-use applicator from a kit comprising a plurality of new single-use applicators. The kit may comprise from about 2 to about 30 new single-use applicators. The single-use applicators in a kit may contain identical compositions or different compositions. In some examples, the insertion portion has an overall length of less than about 60mm, or less than about 50mm, or less than about 45mm. These lengths allow for the insertion portion to treat the vaginal introitus and optionally external vaginal tissue while minimizing the overall size and material required to make the insertion portion. Reducing material may be important if the insertion portion is disposed of after use.

The methods described herein may be directed to and/or performed by women experiencing one or more of vaginal dryness, vaginal irritation, vaginal itch, vaginal infection, vaginal odor, vaginal chafing, dyspareunia, vaginal atrophy, and/or suffering from a reduction in estrogen levels. A reduction in estrogen levels, which may lead to vaginal dryness for example, may be caused by menopause, pregnancy, breast feeding, full hysterectomy, oophorectomy, medical treatments (e.g., chemotherapy, pelvic radiation). In addition to a reduction in estrogen levels, vaginal dryness may also be due to, for example, vaginitis, inflammation of the vagina due to thinning and shrinking of the tissues, sexual arousal disorder, menopause, drug (prescription or over the counter) induced vaginal dryness, dyspareunia, sexual pain disorder, pregnancy, breast feeding, hormone imbalance, anxiety, and diabetes. In addition, the vaginal care compositions may have non-medical uses for females in need of vaginal lubrication. In some methods, the female user may have undergone a treatment for cancer, in which case the vaginal care composition is substantially free from estrogen and/or progesterone agents.

The method may further include a female user holding or grasping the applicator and dispensing from the reservoir of the applicator an amount of the vaginal care composition onto at least a portion of the surface of the insertion portion of the applicator. The method may further include providing a single dose of the vaginal care composition on the insertion portion of the applicator or a portion thereof. The amount of the vaginal care composition dispensed from the applicator may be the single dose sufficient for covering the insertion portion or a lesser amount such that multiple actuations (*e.g*., 2-4) of the applicator are needed to provide a suitable amount.

To treat female genital dryness, vaginal irritation, vaginal itch, vaginal infection, vaginal odor, vaginal chafing, dyspareunia, and/or vaginal atrophy, a female user may grasp or hold the applicator and manipulate the applicator to administer the vaginal care composition to her vaginal introitus and, optionally, one or more external vaginal tissues, using the insertion portion such that at least a portion of the vaginal care composition thereon is transferred to the vaginal tissue. The female user may be seated, standing, laying down, squatting, or have one leg supported by a surface during manipulation of the applicator.

The female user may insert the insertion portion of the applicator into her vaginal introitus using gentle handle pressure (*e.g*., until slight resistance to insertion is noticed by the user) so as not to over-insert the applicator beyond the vaginal introitus and deeper into the vaginal canal. The insertion portion is preferably only inserted far enough to treat the vaginal introitus without discomfort. The insertion portion is inserted only so far as is comfortable to avoid pain or tearing or bleeding from the delicate tissues. The female user may insert the applicator into the vaginal introitus a distance of 40mm, 35mm, 30mm, 25mm or less, or from about 5mm to about 30mm or from about 5mm to about 25mm or from about 5mm to about 20mm or from about 5mm to about 15mm or any range formed by any of the preceding values. In some examples, the fingertip offset distance when the female user grasps the applicator is from about 30mm to 65mm, or from about 30mm to about 60mm, or from about 30mm to about 55mm. Without wishing to be bound by theory, it is believed that consumers may be averse to self-touch and soiling their fingers with bodily fluid and/ or the vaginal care composition. As such, applicators designed to be gripped with the fingertip offset distances described above will result in the insertion portion being inserted minimally into the introitus during use. In some examples, the maximum width of the applicator at a longitudinal distance of 25mm from the tip of the insertion portion is from about 15mm to 45mm, or from about 20mm to about 40mm, or from about 25mm to about 35mm. This may further help limit the insertion of the applicator into the introitus and for a cone-shaped insertion portion provide a slope to the outer surface of the insertion portion that accommodates a wide variety of vaginal introitus and vaginal tissue geometries. In some examples, the maximum width of the applicator where the female user grasps the applicator is from about 20mm to 70mm, or from about 25mm to about 60mm, or from about 30mm to about 55mm. These widths allow for easy grasping and manipulation of the applicator with a single hand during use. In some examples, the insertion portion has an overall length of less than about 60mm, or 50mm, or 45mm. In some examples, the insertion portion has an overall length from about 25mm to about 65mm, from about 25mm to about 55mm, or from about 25mm to about 50mm. These lengths allow for the insertion portion to treat the vaginal introitus and optionally external vaginal tissue while minimizing the overall size and material required to make the removably attached insertion portion. Reducing material may be important if the insertion portion is disposed of after use.

Preferably, the insertion portion is not inserted as far as the middle or upper region of the vaginal canal by the female user. In some examples, the insertion portion is inserted no further than the lower region of the vaginal canal. It is believed that at least some female users may find this method less intimidating and/or more simple and convenient, thereby encouraging long-term habit adoption and compliance. The insertion portion of the applicator may then be used to administer any remaining vaginal care composition thereon (and/or to spread some of the vaginal care composition applied to the vaginal introitus) to the external vaginal tissues, such as labia minora, labia majora, the clitoris, the perineum, the urogenital tract, etc.

In some examples, the shape of the insertion portion (*e.g*., the maximum width Wₘₐₓ of the insertion portion, which may be at the base of the insertion portion) limits the insertion depth of the body when gentle hand pressure is applied to the applicator. Preferably, the tip of the insertion portion is not inserted as far as the middle or upper region of the vaginal canal by the female user. In some examples, the insertion portion or the tip of the insertion portion is inserted no further than the lower region of the vaginal canal. It is believed that at least some female users may find this method less intimidating and/or more simple and convenient, thereby encouraging long-term habit adoption and compliance. The insertion portion of the applicator may then be used to administer any remaining vaginal care composition thereon (and/or to spread some of the vaginal care composition applied to the vaginal introitus) to one or more external vaginal tissues.

The female user may manipulate the applicator by inserting the insertion portion into the vaginal introitus and rotating the applicator using her wrist, retracting the applicator from the vaginal introitus and then swiping the insertion portion along the external vaginal tissues. The tip of the insertion portion of the applicator may trace generally a circular or an oval pattern. In addition, or alternatively, the applicator may be rotated back and forth about its longitudinal axis. In addition, or alternatively, the female user may manipulate the applicator by pivoting the applicator up and down about the wrist or fingertips. The female user may further manipulate the applicator by swirling, pivoting, swiping, rotating, revolving, or a gliding motion through use of her fingertips or wrist. In certain methods, the aforementioned manipulations may be applied to the vaginal introitus and optionally one or more external vaginal tissues. In certain methods, a combination of the aforementioned motions may be performed.

In some examples, the female user may administer the vaginal care composition to the vaginal tissues of interest for a period of time from about 1 second to about 40 seconds, or from about 1 second to about 30 seconds, or from about 1 second to about 15 seconds or any range formed by any of the preceding values. A short administration time is desirable for providing a method that is convenient and quick to complete.

Because the experience of both the applicator and vaginal care composition may be useful for encouraging long-term habit adoption by a female user for both acute and chronic treatment of one or more vaginal conditions, such as vaginal dryness, vaginal irritation, vaginal itch, vaginal infection, vaginal odor, vaginal chafing, and/or vaginal atrophy, the female user may use the applicator to apply the vaginal care composition on a regular basis versus as a mere precursor to sexual intercourse. With respect to frequency of administration, in some examples, the method may be performed by a female user at least twice per week or three, four, five, six times per week, or more for a period of at least 4 weeks, or 8 weeks, or 12 weeks, or more. In other instances, the method may be performed daily for a period of at least 4 weeks, or 8 weeks, or 12 weeks, or more, optionally with a plurality of applicators over the treatment period. In some instances, the female user may perform the method for 6, 8, 10, 12 months or more. In some instances, the female user may use the same insertion portion every time she performs the method. In other instances, the female user may use a new insertion portion each time she performs the method. In other instances, the female user may use the same insertion portion for some number of times and then use a new insertion portion (e.g., perform the method for 4 weeks using a new insertion portion each week). The female user may clean the insertion portion between repeating the method. The user may perform the method more than once a day (e.g., morning and evening). The method may be performed by a female user at about the same time each day or following a daily ritual activity in order to facilitate habit adoption and habit compliance. For example, the female user may perform the method in the morning, as part of a routine (*e.g*., after showering). Similarly, a female user may perform the method in the evening before bed or at any other time convenient to the female user.

The methods may further include cleaning or washing the applicator so that the applicator may be reused at a later time to repeat administration of the vaginal care composition. The applicator may be sufficiently durable and/or hygienic so that the female user may safely repeat the method 2, 5, 7, 10, 14, 20, 30, 40, or 50 times or more using the same applicator. The reservoir of an applicator may be refillable or replaceable. The applicator may be disposable after a single use or the applicator may store enough of the vaginal care composition for multiple uses. For an applicator that comprises a removable insertion portion, the insertion portion may be disposable after a single use

While it may be desirable for the applicator to be durable, it is appreciated that daily use may result in some degradation or soiling over time and require replacement of the applicator (or certain parts, like the insertion portion) by a new or fresh applicator after a single use or 1, 2, 3, or 4 weeks or more of use. The new or fresh applicator may be obtained separately or as part of a new kit that includes a new applicator and/or body comprising a vaginal care composition. The female user may use from about 4, 6, 12, or more fresh or new applicators/insertion portions per year. In some examples, the applicator may be rinsed, sprayed, or immersed in a liquid and/or wiped with a substrate to remove any residual vaginal care composition or bodily fluids.

### D. Test Procedures

### i. Applicator Surface Texture Method

In the Applicator Surface Texture Method, the areal surface topology of the applicator is measured using optical profilometry. The 3D surface data are processed to measure the microscale areal surface roughness parameters Sq (root mean square height), as described in ISO 251782:2012, which characterize the surface roughness over a given surface area. All sample preparation and testing is performed in a conditioned room maintained at about 23 °C ± 2 °C and about 50% ± 2% relative humidity, and samples are equilibrated in this environment for at least 24 hours prior to testing.

### Sample preparation

Five new substantially similar replicate applicator samples are selected for analysis.

### 3D surface image acquisition

Three-dimensional (3D) surface topography images of the applicator area of interest (*e.g*., the tip, the grippable portion) are recorded using an optical 3D surface topography measurement system (a suitable optical 3D surface topography measurement system is the MikroCAD Light instrument commercially available from LMI Technologies Inc., Vancouver, Canada, or equivalent). The system includes the following main components: a) a Digital Light Processing (DLP) projector with direct digital controlled micro-mirrors; b) a charge coupled device (CCD) camera with at least a 1600 x 1200 pixel resolution; c) projection optics adapted to a measuring area of at least 5 mm x 4 mm; d) recording optics adapted to a measuring area of 5 mm x 4 mm; e) a table tripod based on a small hard stone plate; f) a blue LED light source; g) a measuring, control, and evaluation computer running surface texture analysis software (a suitable software is MikroCAD software with MountainsMap technology, or equivalent); and h) calibration plates for lateral (xy) and vertical (z) calibration available from the vendor.

The optical 3D surface topography measurement system measures the surface height of a sample using the digital micro-mirror pattern fringe projection technique. The result of the measurement is a 3D image of surface height (defined as the z-axis) versus displacement in the horizontal (xy) plane. The system has a field of view of 5 mm x 4 mm with an xy pixel resolution of approximately 3 microns, and a height resolution of 0.1 microns, with a height range of ± 2 mm.

The instrument is calibrated according to manufacturer's specifications using the calibration plates for lateral (xy plane) and vertical (z-axis) available from the vendor.

The applicator sample is placed on the table such that the region to measure is oriented horizontally beneath the camera. Analysis regions are selected such that the curvature within the region imaged is minimized. Each analysis region excludes any visually obvious macroscopic features such as an: opening, ridge, groove, edge, or corner.

A 3D surface topology image of the sample is collected by following the instrument manufacturer's recommended measurement procedures, which may include focusing the measurement system and performing a brightness adjustment. No pre-filtering options are used. The collected height image file is saved to the evaluation computer running the surface texture analysis software.

### 3D surface image analysis

The 3D surface topography image is opened in the surface texture analysis software. The following filtering procedure is then performed on each image: 1) a 5x5 pixel median filter to remove noise; 2) a 5x5 pixel mean filter to smooth the surface; and 3) a Gaussian filter (according to ISO 16610-61) with a nesting index (cut-off) of 0.8 mm without utilizing end effect correction to flatten the surface; 4) Threshold and remove the heights corresponding to a material ratio value of less than 2% and a material ratio of greater than 98% to remove outliers. The Areal Material Ratio (Abbott-Firestone) curve, described in the ISO 13565-2:1996 standard and extrapolated to surfaces, is the cumulative curve of the surface height distribution histogram versus the range of surface heights. A material ratio is the ratio, given as a %, of the intersecting area of a plane passing through the surface at a given height to the cross-sectional area of the evaluation region.

This filtering procedure produces the surface from which the Sq values, as described in ISO 25178-2:2012, are calculated. Record the surface roughness values for Sq to the nearest 0.1 µm. Repeat this procedure over the same area of interest for the remaining replicate samples. The surface texture is the average of the 5 replicate Sq measures to the nearest 0.1 µm.

### ii. Viscosity

The viscosity of samples is measured using Brookfield RV viscometer fitted with a helipath T-bar spindle. The viscometer is leveled, setup and calibrated according to the manufacturer's standards. The viscometer speed (RPM) and spindle (e.g., Type T-A, T-B, T-C etc.) are selected to ensure the measured viscosity is within the manufactures recommended settings.

Samples are stored in sealed glass jars with an opening and internal diameter of at least 40mm and filled to a height of at least 50mm with care taken to avoid entrapped air bubbles. Centrifugation may be used to help remove entrained air. Sample jars are equilibrated at 23 °C ± 2 °C and about 50% ± 2% relative humidity for at least 24 hours prior to measurement.

Viscosity is measured at 23 °C ± 2 °C and about 50% ± 2% relative humidity by placing the uncapped sample jar under the viscometer and lowering the viscometer until the tip of the T-bar touches the surface of the sample. The descending helipath is turned on and a timer started once the cross-bar of the T-bar touches the surface of the sample. For 1 minute, a reading is taken every 10 seconds. The viscosity is the arithmetic average of the viscosities recorded. Care is taken to ensure the T-bar does not touch the glass jar.

## Claims

1. An applicator (100) for treating one or more vaginal tissues, comprising:
a tapered, insertion portion (150) comprising one or more dispensing openings (152);
a body (110) disposed adjacent the insertion portion (150) comprising a reservoir (102) in fluid communication with the one or more dispensing openings (152), wherein the reservoir (102) stores greater than 10g to about 200g of a vaginal care composition.

2. The applicator (100) according to any one of the preceding claims, wherein the reservoir (102) is collapsible, preferably wherein the applicator (100) further comprises a cavity (307) surrounding the collapsible reservoir (102) and a vent (312) in communication with the cavity (307).

3. The applicator (100) according to any one of the preceding claims, wherein the body (110) comprises a sleeve (120) or a tube and an elevator (118) slideably disposed within the sleeve (120) or tube.

4. The applicator (100) according to claim 3, wherein the applicator (100) further comprises a rotatable screw that engages the elevator (118).

5. The applicator (100) according to any one of the preceding claims, wherein the fingertip offset distance is from about 30mm to 65mm, or from about 30mm to about 60mm, or from about 30mm to about 55mm.

6. The applicator (100) according to any one of the preceding claims, wherein the applicator (100) further comprises a means for reducing backflow in fluid communication with the reservoir (102).

7. The applicator (100) according claim 6, wherein the one or more dispensing openings (152) are in fluid communication with the means for reducing backflow.

8. The applicator (100) according to any one of the preceding claims, wherein the applicator (100) comprises from about 2 to about 50 dispensing openings (152) disposed about the insertion portion (150).

9. The applicator (100) according to any one of the preceding claims, wherein the one or more dispensing openings (152) having an opening area from about 0.05mm² to about 80mm².

10. The applicator (100) according to any one of the preceding claims, wherein the applicator (100) has an overall length from about 35mm to about 175mm.

11. The applicator (100) according to any one of the preceding claims, wherein the insertion portion (150) is removably attached to the body (110).

12. The applicator (100) according to any one of the preceding claims, wherein the vaginal care composition has a viscosity from about 2,000 cps to about 200,000 cps.

## Patentansprüche

1. Applikator (100) zum Behandeln eines oder mehrerer Vaginalgewebe, umfassend:
einen konischen Einführabschnitt (150), umfassend eine oder mehrere Abgabeöffnungen (152);
einen Körper (110), der benachbart an den Einführabschnitt (150) angeordnet ist, umfassend einen Behälter (102) in Fluidaustausch mit der einen oder den mehreren Abgabeöffnungen (152), wobei der Behälter (102) mehr als 10 g bis etwa 200 g einer Vaginalpflegezusammensetzung speichert.

2. Applikator (100) nach einem der vorstehenden Ansprüche, wobei der Behälter (102) zusammenklappbar ist, vorzugsweise wobei der Applikator (100) ferner einen Hohlraum (307) umfasst, der den zusammenklappbaren Behälter (102) umgibt, und eine Entlüftungsöffnung (312), die mit dem Hohlraum (307) in Verbindung steht.

3. Applikator (100) nach einem der vorstehenden Ansprüche, wobei der Körper (110) eine Hülse (120) oder eine Röhre und einen Heber (118) umfasst, der verschiebbar innerhalb der Hülse (120) oder der Röhre angeordnet ist.

4. Applikator (100) nach Anspruch 3, wobei der Applikator (100) ferner eine drehbare Schraube umfasst, die in den Heber (118) eingreift.

5. Applikator (100) nach einem der vorstehenden Ansprüche, wobei der Fingerspitzenversatzabstand von etwa 30 mm bis 65 mm oder von etwa 30 mm bis etwa 60 mm oder von etwa 30 mm bis etwa 55 mm beträgt.

6. Applikator (100) nach einem der vorstehenden Ansprüche, wobei der Applikator (100) ferner ein Mittel zum Reduzieren von Rückfluss in Fluidaustausch mit dem Behälter (102) umfasst.

7. Applikator (100) nach Anspruch 6, wobei die eine oder die mehreren Abgabeöffnungen (152) mit den Mitteln zum Reduzieren von Rückfluss in Fluidaustausch sind.

8. Applikator (100) nach einem der vorstehenden Ansprüche, wobei der Applikator (100) von etwa 2 bis etwa 50 Abgabeöffnungen (152) umfasst, die um den Einführabschnitt (150) herum angeordnet sind.

9. Applikator (100) nach einem der vorstehenden Ansprüche, wobei die eine oder die mehreren Abgabeöffnungen (152) eine Öffnungsfläche von etwa 0,05 mm² bis etwa 80 mm² aufweisen.

10. Applikator (100) nach einem der vorstehenden Ansprüche, wobei der Applikator (100) eine Gesamtlänge von etwa 35 mm bis 175 mm aufweist.

11. Applikator (100) nach einem der vorstehenden Ansprüche, wobei der Einführabschnitt (150) an dem Körper (110) entfernbar angebracht ist.

12. Applikator (100) nach einem der vorstehenden Ansprüche, wobei die Vaginalpflegezusammensetzung eine Viskosität von etwa 2.000 cPs bis etwa 200.000 cPs aufweist.

## Revendications

1. Applicateur (100) destiné à traiter un ou plusieurs tissus vaginaux, comprenant :
une partie d'insertion effilée (150) comprenant une ou plusieurs ouvertures de distribution (152) ;
un corps (110) disposé adjacent à la partie d'insertion (150) comprenant un réservoir (102) en communication du point de vue des fluides avec l'une ou plusieurs ouvertures de distribution (152), dans lequel le réservoir (102) stocke plus de 10 g à environ 200 g d'une composition de soins vaginaux.

2. Applicateur (100) selon l'une quelconque des revendications précédentes, dans lequel le réservoir (102) est pliant, de préférence dans lequel l'applicateur (100) comprend en outre une cavité (307) entourant le réservoir pliant (102) et un évent (312) en communication avec la cavité (307).

3. Applicateur (100) selon l'une quelconque des revendications précédentes, dans lequel le corps (110) comprend un manchon (120) ou un tube et un élévateur (118) disposé de manière coulissante au sein du manchon (120) ou du tube.

4. Applicateur (100) selon la revendication 3, dans lequel l'applicateur (100) comprend en outre une vis rotative qui vient en prise avec l'élévateur (118).

5. Applicateur (100) selon l'une quelconque des revendications précédentes, dans lequel la distance de décalage de bout de doigt est d'environ 30 mm à 65 mm, ou d'environ 30 mm à environ 60 mm, ou d'environ 30 mm à environ 55 mm.

6. Applicateur (100) selon l'une quelconque des revendications précédentes, dans lequel l'applicateur (100) comprend en outre un moyen destiné à réduire le reflux en communication du point de vue des fluides avec le réservoir (102).

7. Applicateur (100) selon la revendication 6, dans lequel l'une ou plusieurs ouvertures de distribution (152) sont en communication du point de vue des fluides avec le moyen destiné à réduire le reflux.

8. Applicateur (100) selon l'une quelconque des revendications précédentes, dans lequel l'applicateur (100) comprend d'environ 2 à environ 50 ouvertures de distribution (152) disposées autour de la partie d'insertion (150).

9. Applicateur (100) selon l'une quelconque des revendications précédentes, dans lequel l'une ou plusieurs ouvertures de distribution (152) ayant une surface d'ouverture d'environ 0,05 mm² à environ 80 mm².

10. Applicateur (100) selon l'une quelconque des revendications précédentes, dans lequel l'applicateur (100) a une longueur globale de 35 mm à 175 mm.

11. Applicateur (100) selon l'une quelconque des revendications précédentes, dans lequel la partie d'insertion (150) est fixée de manière amovible au corps (110).

12. Applicateur (100) selon l'une quelconque des revendications précédentes, dans lequel la composition de soins vaginaux a une viscosité d'environ 2 000 cps à environ 200 000 cps.
